# FASCICULE DE BREVET EUROPEEN

(11) **EP 1 335 646 B1**
(45) Date de publication et mention de la délivrance du brevet: **17.01.2007**
(21) Numéro de dépôt: 01997219.9
(22) Date de dépôt: 23.11.2001
(51) Int. Cl.: A01K 67/027, C07K 14/47, C12N 15/12, C12N 15/85, C12Q 1/68, A61K 49/00, G01N 33/50

(54) **MAMMIFERES NON-HUMAINS TRANSGENIQUES OU RECOMBINANTS POUR LA PROTÉINE STOP ET LEURS APPLICATIONS DANS LE CRIBLAGE DE MEDICAMENTS PSYCHOACTIFS**
NICHT-MENSCHLICHE TRANSGENE ODER REKOMBINANTE SÄUGETIERE FÜR DAS STOP PROTEIN UND VERWENDUNGEN DAVON ZUR AUSWAHL VON PSYCHOAKTIVEN ARZNEIMITTELN
TRANSGENIC OR RECOMBINANT NON-HUMAN MAMMALS FOR STOP PROTEIN AND THEIR USES IN SCREENING PSYCHO-ACTIVE MEDICINES

(30) Priorité: 24.11.2000 FR 0015240
(43) Date de publication de la demande: 20.08.2003
(73) Titulaire: COMMISSARIAT A L'ENERGIE ATOMIQUE, 75015 Paris (FR); INSTITUT NATIONAL DE LA SANTE ET DE LA RECHERCHE MEDICALE (INSERM), 75654 Paris Cédex 13 (FR)
(72) Inventeur: ANDRIEUX, Annie, F-38100 Grenoble (FR); JOB, Didier, F-38100 Grenoble (FR); DENARIER, Eric, Montréal H2J 3G3 (CA); BOSC, Christophe, F-38000 Grenoble (FR); VERNET, Muriel, F-38000 Grenoble (FR)
(74) Mandataire: Vialle-Presles, Marie José
(86) Numéro de dépôt international: PCT/FR2001/003701
(87) Numéro de publication internationale: WO 2002/041691

(56) Documents cités:
- WO-A-00/34336
- MARTINDALE: "The Complete Drug Reference" 1999 , PHARMACEUTICAL PRESS , LONDON XP002200547 page 649 - page 668; page 673- page 675
- DATABASE BIOSIS [en ligne] BIOSCIENCES INFORMATION SERVICE, PHILADELPHIA, PA, US; 2001 ANDRIEUX A ET AL: "Behavioral disorders and impairment of synaptic plasticity in stop deficient mice." Database accession no. PREV200100490973 XP002200548 & SOCIETY FOR NEUROSCIENCE ABSTRACTS, vol. 27, no. 1, 2001, page 725 31st Annual Meeting of the Society for Neuroscience;San Diego, California, USA; November 10-15, 2001 ISSN: 0190-5295
- GUILLAUD L ET AL: "STOP proteins are responsible for the high degree of microtubule stabilization observed in neuronal cells" J CELL BIOL, vol. 142, no. 1, 13 juillet 1998 (1998-07-13), pages 167-179, XP002178084 cité dans la demande
- DENARIER E ET AL: "Genomic structure and chromosomal mapping of the mouse STOP gene (Mtap6)" BIOCHEM BIOPHYS RES COMM, vol. 243, no. 3, 24 février 1998 (1998-02-24), pages 791-796, XP002178083 cité dans la demande
- DENARIER E ET AL: "Nonneuronal isoforms of STOP protein are responsible for microtubule cold stability in mammalian fibroblasts" PROCEEDINGS OF THE NATIONAL ACADEMY OF SCIENCES OF USA., vol. 95, no. 11, 26 mai 1998 (1998-05-26), pages 6055-6060, XP002178082 cité dans la demande
- GOUDET H ET AL: "Perikaryal aggregation of STOP proteins analyzed in a transgenic model" JOURNAL OF NEUROCHEMISTRY, vol. 74, no. Suppl., 26 mars 2000 (2000-03-26), page S30 XP001024412
- PEI Q ET AL: "Changes in mRNA abundance of microtubule-associated proteins in the rat brain following electroconvulsive shock" NEUROREPORT, vol. 9, no. 3, 16 février 1998 (1998-02-16), pages 391-394, XP001024411

### LISTE DE SEQUENCES

<110> COMMISSARIAT A L'ENERGIE ATOMIQUE
   INSTITUT NATIONAL DE LA SANTE ET DE LA RECHERCHE MEDICALE-INSERM
   ANDRIEUX Annie
   JOB Didier
   DENARIER Eric
   BOSC Christophe
   VERNET Muriel
<120> MAMMIFERES NON-HUMAINS TRANSGENIQUES OU RECOMBINANTS ET LEURS APPLICATIONS DANS LE CRIBLAGE DE MEDICAMENTS PSYCHOACTIFS.
<130> SEQ263FR63
<140>
   <141>
<160> 6
<170> PatentIn Ver. 2.1
<210> 1
   <211> 7133
   <212> ADN
   <213> Mus musculus
<400> 1
   gaattcaaag atgccattat cttgctttct ttcgtcccta cttctgatct cacggcagcc 60
   ccagcagagg tcctatgagt cctccaagga ctacgtagct tcaacactga agcctgtaac 120
   tttgtcccat ggtcccctaa tgcgtaagga aaaagcccag catttcacga taaagcagga 180
   agcagagtga gtgcctttga tataataggg aaggtctaac ttacaggcta ttttaagaat 240
   tgattgctaa gtatcaatat gatcacgctt ttaagatgac tcatgatctt ccacattaat 300
   ctgttgtgtg tggaattata tctgtacatt gctggctatt aaaccatggc ttgctcctaa 360
   cataaacact actaaatatc agatttttct atctatctat ctatctattg atctatctat 420
   ctatctatct atctatctac ctacctacct acctatcatc tatctatcta tcaatcatgt 480
   atgtatgtat gtatgtatgt atgtatctat ctatctatct atctatctaa gtgtgtatga 540
   gtgtagctgg gcataacatc acatgcttgt atgtagaggt aggaggacaa tcccttgaac 600
   agatccctca tcaacaagtt ggatctggcc aaattggcat aagaaaaaaa aagctcaatg 660
   tattatgttg ctaaagactt gcaaattaac aacaaatgta acccaaacag acacagcacc 720
   aaacactggc aagggggaga gaaacaggag ttctcattag ttactactga tcactcaaaa 780
   ctggtacaac tatggtggaa gacagtttgt tttgttacaa aactaactca tcagttacta 840
   ctgagtactc aaaactggta caattatggt ggaagacagt ttgctttgtt acaaaactag 900
   ctattctttt ggccacataa tcacaatcct gctccttgat attacccaaa aagagcccaa 960
   agcttatgtc agcacagaaa cctgaacact gttttagcag ctttgtttat aattcccccc 1020
   aattgaagca accagcatgc cattcagcag gtgacggata aataatctat gggaacacgt 1080
   aatattacag aacaagagaa atgagatacc agccatgaaa agatatgatg gatccttgag 1140
   cttattacta agtcagagct aatctgaaac actgcttact atctgatgga tcaaaaagga 1200
   ggaatctacc tcaggtgaca agatgaacct tgtcacctcc ttaagggtta atccagtgag 1260
   acaggtgagg ggactgagag gatccacccc aggaaagaca tattcacctt acaataacgt 1320
   actcttccct tacagaatta tggcctccta atgcagttcc cttgctatgc aagtgtgagc 1380
   acatgagttc aagtcctgca cagtgaagca caaatcaggg tgtggtcagg tgagcacctg 1440
   ggacctcagc actgtgaggg gcagagccag gaaaactgca ggggcttgct agccagtgag 1500
   cctagcttca gattcagtga gaaaccttgt ttcatgggga aaaaggcaga gaatggtaga 1560
   gcagaatacc cagcatcctc ccttagacgt acacatgtgc atatatgcca caaataaatt 1620
   aattaagagt aaatatttgg gacttttaag aaaatgaact ctttcttttc cttgtgaagt 1680
   agcagatttc caagtagcag gcttctcagt ggcttatctg ggaggctctg cctgtgagac 1740
   ttttggataa gcttttgaat attaacaggt tctgctgaag aagccatttc tatatgccag 1800
   gagctgcagc taatatttct tgtgaattgt gtcacttcag tcttcagaaa aactttgtaa 1860
   gttcagaata gatacccttg ctcagagaca aagttaaggg tcagaggttg ccacaatttg 1920
   tctatgcctt agagccaaaa gtgcagaagc caggatccaa atccctgagt atggactcca 1980
   ggcttctgct ctgggtgctt aactttcctc atgaaaccac cccgaggcat ttattagctc 2040
   tactaccctc ccccccccca ccccccgccc cgacccaggg gactgcagcc cagagacaac 2100
   ccaagtcaca aagagagtat cagagccaaa gcaggtccat tagattccaa aatctgttca 2160
   accccaaagt agaaaagaag gcagaaacac aaccgagaat cccctcaaag aggtctccct 2220
   tttcctaggg aactgtctct catccagccg cttacctcct cccttgggtc tctttccaga 2280
   atcttccttt ttctcccttt cccccagtgc agcatctggg ctggagaaga accaatttta 2340
   tcctgggaga gtagactcac tgcttcccag ttgcttaaag aaggaacagc cagagtatgg 2400
   ctaggccggc ttcccaggcc ggcctctccc aatctcatac aactggggaa gcccctgtac 2460
   acataggctt aggtcaagaa ccagcaacac ggagcataag tacagcctga tagctgacca 2520
   attaactgca gctctccctt ttaaagtatc tattcagggg ctgagtggtt aaaaacttgt 2580
   attggtgttc cagaggaccc aagtccgggt cccagcattc acatgggggt ggggtgggag 2640
   ctcatagtcg cctgcacagc accagggaaa tctacttctg accctctggc ctcagcaggc 2700
   accagagccc atgtgcatat acccaacaca gacaaaagca ctaattaaaa ggtggcgcaa 2760
   ccctttaatc ccagcacttg ggaggcaaag gcaagcggct tttctgagtt caaggccagc 2820
   ctggttacag actgagttcc aggacagcca agactacaga gagaaaccct gttcgaaaaa 2880
   acaaaagcca aaaaccaaaa aaaaaaaaaa gtaaaaaaga tttttaaaag aagccaaagc 2940
   atttagtctg aaacagacat ttcatggagg cggggcggtc ttacacttaa aaaattagta 3000
   ttcattggtt ggggcgattt tacgaaagag aggagactcg gttttaggac atgaagctgg 3060
   caactcaagg acagcaagtc ctcaggagct tgggacttga acctatcaag gacccagttc 3120
   tgctgttgcc tgggaacaga gaagggagga gctgccagag agaagggagg aggggacagg 3180
   gagagggcaa cctttcagct gcgcgccctg acgacagcag gtgatttttt ttttttttct 3240
   gcagcgcgca tactctcagc atcttttctt gatcacccca cctcccttgc tgagccgcaa 3300
   aaaagggtgg ggccgcctct ctgcagcaga agacgacagc ggcagcggct gcggcatcac 3360
   cgggggtata gatagaggcc gttttgctct ctgctctgtc tgggttggag gtgaccgctg 3420
   ccaagcctcg ctaggcggcc ggctgaacca gacagaaagg agataaaagc ctgcttgcga 3480
   tccttcctgc gccatggctt aagcccacag cctctttgcc aagcatctcc ttgctctgcc 3540
   ggggtctgct agacaccgca gtcgcagaga gggcgcgccc agacgcccta ggcctggact 3600
   ctgggacgct gagcctcgct cctattcttc actgcccaca gcagctcctc tgcagcaggc 3660
   gtttgcagcc ggcaatcgag ggactttacg gactttatct cagcggtacc ttgtccccgg 3720
   gtgctctttg agggtggagg acgaggcaaa gggcttctaa gggaaggaag cggtgggaac 3780
   cacattggcg ggtctgggtt ggggttaaag ggagattgga gatttgattt aggaccacaa 3840
   aaaggctttg tggctaacat ggcgtggccg tgcatcacaa gggcctgctg catcgcccgc 3900
   ttctggaacc agctggacaa ggcggacatt gcggtgccgc tggttttcac caagtactcg 3960
   gaggccaccg aacacccagg cgcccctccg cagccgccag ctccgctgca gcccgcgtta 4020
   gcgcccccct cgcgtgctgt cgccatagag acgcagccag cccagggaga gtcggatgca 4080
   gttgcccggg caacagggct tgcgcccggg cccagcgtcg accgcgagac tgtagccgcc 4140
   cccggccgga gcgggctggg cttgggcgcg gcctcagcct ccacttccgg ctcaggcccc 4200
   gcggactcgg tgatgcgaca ggactaccgc gcctggaaag tgcagcggcc cgagccaagc 4260
   tgccggccgc gcagcgagta ccagccgtcc gacgcgccct tcgagcgcga gacccagtac 4320
   cagaaggact tccgcgcctg gccgctgccc cggcgcgggg accatccctg gatccccaag 4380
   ccggtgcaaa tccctgcgac ttcgcagcct tcccaacctg ttctcggggt gcccaagcgt 4440
   cggcctcaga gccaagagcg cgggcccatg caactttctg ctgatgcccg ggacccggag 4500
   ggtgctggag gagccggggt gctggcggca ggaaaggcgt ccggtgtaga ccagcgcgac 4560
   acacgtagga aggcagggcc agcatggatg gtgactcgca acgaagggca cgaagagaag 4620
   cctctgcccc cagcccaatc ccagacccag gagggtggtc ctgcagctgg aaaggcgtcc 4680
   ggtgcagatc agcgtgacac acgcaggaag gcagggccag catggatggt gactcgcagc 4740
   gaagggcacg aagagaagcc tctgccccca gcccaatccc agacccagga gggtggtcct 4800
   gcagctggaa aggcgtccgg tgcagatcag cgtgacacac gcaggaaggc tggacccgcg 4860
   tggatggtga ctcgcacgga agggcacgag gagacgccgc tgccacccgc ccagtctcag 4920
   acccaggagg gcggccccgc agctggaaag gcatctggtg cagacgagcg cgacacgagg 4980
   aggaaggcgg ggccggcctg gatggtgcgt cgctcggagg ggcacgaaca gacacccgct 5040
   gcccatgccc aaggcacagg gcctgaagga agcaaggggc gcgcggtggc agatgccctc 5100
   aacaggcaaa tccgggagga ggtggcgagt acagtaagca gctcttacag gtgagactgg 5160
   ggcagcaggt gatgctggtc accctcatcc cctcgcgagg accacccatt ctacccccac 5220
   accgaaagct tcgcattcag cttcttctcc agggccagac cacacctctt cagccacatt 5280
   ccagaaccct ttcaacccag actttactgc ccaccctgtc ggaaagccct taacagtttc 5340
   cacactggtt ttcccagctt gttttttgtg ccacccctaa gccacatttt cctcttggct 5400
   gcctagctca gctccctatc tgccccacag agaccctgtc agtttccccc tgtctcatca 5460
   gttcgcctgc tgtcccagcc tggctcccgt ccctagtcgc ctccactcat tcacttctca 5520
   cttacttccc cgtgagaccc tttctcctcc ccagtctcac attgtcctgg tctcttccta 5580
   ttgattcctc ccccagttct ggccgacact caagcgccac acccttttcc agatttctct 5640
   acatgctcct taataactgg ctctagtact tgaagtcatt cccctcctgc ctcttcagta 5700
   gctttgacac attgggcgag ctttttaacc ttcctgggcc ttcacattct tatctgtaac 5760
   tgggatcaat aatagtcaac ttagaactat ccacacagga ctgatttaag ctgagatatt 5820
   ctgtacagtt ttgaggatag actaaatgcc aaacatggct ctagctgatg gaaagtggaa 5880
   agagatgact cgggatgact ggccatgcct gagatgtagt gagaggtctt gtttccattt 5940
   atactctcct atgtgatgcc cccttattct tgattctgct tcctggatgt ccctagccct 6000
   cttccctgtt gaggcagctc ctttcttcct ttgtgttcag atgtttctat tttttctttt 6060
   aattaattag ttaattaatt aatttactta ttccctttac atcctgctca ctgcccccta 6120
   cagtccttct ccccatgccc cacttcttct ctaagtgggt ggggtctccc cctgagtatc 6180
   tccctaccct ggtacatcaa gtttctgcag ggctagtcta atcctctccc actcaggcca 6240
   aacaagtcaa cccagctaga agaacatatt ccacgaacag ccaacagctt tttggggtag 6300
   cccccattcc agttgtttgg gacccacatg aagaccaagc tgcacatctg ctgtatatgt 6360
   gcagagaggt ctaggtccag cccatgtatg ctctttggtt tgtggttcag tctctgagaa 6420
   cccctaaggg ttccaggtta gttgactctg ttgatcttcc tgtggagttc ctatctcctt 6480
   aggggcctac aatccttctc cctatacttc cacaagactc cccaagctcc atccactgtt 6540
   tggccatggg tgtctgtatc tgtctgagtc agctgctggg tggagcttct caaaggtcag 6600
   acattaatgt gtcccttatc ctctggcaca ttcacgttcc ttgaaaatga acgctttcaa 6660
   acttagaaac atcccccatc ttcctccacg cacaccctgt ttatacaatt ctccctgtgc 6720
   tgcatgcagc cttggcctgc tttctcacct cacatccatc cagtgcacat ctctgaatgt 6780
   ttagcttttg ggccttcctt tatgtttcta ccaacatcag ctagctgata gatcctagtt 6840
   gacttgaaca tgtagcagca ccgggcttgg gtactgagga agcagatgtg tgtgataatg 6900
   atggagctgg ccttgtgcag ctgtgacttc tgaggtcttc attcagctcc acagtcagtt 6960
   aggtgccctt ttcctaagtg tggaaggaag ggctacagag ttagtgtctt ctgctaaccc 7020
   tgtgtgtggg cgcttcagta ggctctgcag gcactcctct catgcagcca gaagactggc 7080
   ataaatagag actctattgt taaggcaaaa gtcagagccc tgcctcagaa ttc        7133
<210> 2
   <211> 1881
   <212> ADN
   <213> Mus musculus
<400> 2
   ctgtgagttg gtggccagct cggtttacat agtgagttcc aggacagcca gggcagaaaa 60
   aaaaaaaaag tacctatata aaacttatga ggggctggtg agatggctca gtgggttaga 120
   gcacccgact gctcttccga aggtccagag ttcaaatccc agcaaccaca tggtggctca 180
   caaccatccg taatgagatc tgactccttc ttctggagtg tctgaagaca gctacaatgt 240
   acttacatat aataaataaa taaataaata aataaatctt aaaaaaaaaa acttatgaga 300
   acatcggcgg agggacagtc ccttaaagag ccatttgtta tagctgaaga gatggtttca 360
   ctggatagca gcacttgctg ctcttgcaga gaacccaggc tcagttcctc atccctatat 420
   gatggttcgc aaccatttgt aactccagtt ccaggggaac caacgatacg tgtacataca 480
   agcaggcaaa acacccacac tcataaaatg aaatacacaa ctcttaactg tttttgttca 540
   cctgtctcca cccccagtgt tggggtagca gacacatgca gctatgccta acttttacgt 600
   aagttctggg ggcctgaatt ccaatcttca tcccgatcca gcaagcactt ttacccctgt 660
   agccatctcc ttggccttga gaaagcttct taagggttga tatcattccg aggaaaatat 720
   tgaatacata tttactatgg agtcatgtat atttaagaaa tgcacacagc atgcatttat 780
   gcctccttcc acgttctgga aacactggag gaaggtaggc agggagtgtg tttaggatgg 840
   aatttaaaat ttatccgggc cattttattt aagggcatct cagatctatc accaaggatt 900
   cccacttaga aaatttggac aacataagcc tcaaaatgaa aagtgaaaaa aatgggttaa 960
   taccacagta aaaatattca tgaatccgac agtgtataac aaagaggcca gctactaagt 1020
   gtagaaagaa taaaggattc tgagaaatca tcatgtgaca aacatcatca tagcgagtgt 1080
   gctgggcaag ggacgccagc agttggtaaa tgatcgggcg acaattcctt gggtaacaaa 1140
   caaacgagtt gcagagtgtc aggtgacctt gcttagtgta gtagttggac tggataatga 1200
   atagcagagc accttgaaat ctgaggacgt tatagttgtt ttaaaaatgt cttcacaagg 1260
   ccctcatgtt gtgtaaagga gattttaaaa ttttagttca acaaaaagaa gacatgttta 1320
   ctgtgtatct tagcctgttt tagttctggg acagacacca tagataccga gctgaaacaa 1380
   caagtgctct ttacccacag ttccagggcg tgggaagtac aacatcaaag ctctgatgca 1440
   agtggtgtcc gtgaggatcc atttcctgct atgcagatgc tgcaaacata tgactccggc 1500
   ctcttcctct tctttaaggg catgtgtctg aagtggggtg gggtggcatc ctcatgactt 1560
   catctgtgtt ttccctagag atagcccctt tggttagggt tttaacatag atgtttggtg 1620
   cacacataca tttagtctat gatagtctct agcaacagcc agttgggagg gaaggagagg 1680
   gagagggaga gagagagaga gagagaacac atgtatgcga gtctgtgatt tctagcttct 1740
   tagcttctta tatttgatct aattttcatt tattttatga ttctggaact ctgttgtttt 1800
   attactttga aattctagga ctccaaataa atatttttga tcttaaaata ttatcattat 1860
   tttattctga tcccagaatt c                                           1881
<210> 3
   <211> 27
   <212> ADN
   <213> Séquence artificielle
   <220>
   <223> Description de la séquence artificielle:amorce oligonucléotidique
<400> 3
   agagtcggat gcagttgccc ggcaaca 27
<210> 4
   <211> 27
   <212> ADN
   <213> Séquence artificielle
<220>
   <223> Description de la séquence artificielle:amorce oligonucléotidique
<400> 4
   ggctcctcca gcaccctccg ggtcccg 27
<210> 5
   <211> 21
   <212> ADN
   <213> Séquence artificielle
<220>
   <223> Description de la séquence artificielle:amorce oligonucléotidique
<400> 5
   aacagggtgt gcgtggagga a 21
<210> 6
   <211> 1183
   <212> ADN
   <213> Mus musculus
<400> 6
   gatatcattc cgaggaaaat attgaataca tatttactat ggagtcatgt atatttaaga 60
   aatgcacaca gcatgcattt atgcctcctt ccacgttctg gaaacactgg aggaaggtag 120
   gcagggagtg tgtttaggat ggaatttaaa atttatccgg gccattttat ttaagggcat 180
   ctcagatcta tcaccaagga ttcccactta gaaaatttgg acaacataag cctcaaaatg 240
   aaaagtgaaa aaaatgggtt aataccacag taaaaatatt catgaatccg acagtgtata 300
   acaaagaggc cagctactaa gtgtagaaag aataaaggat tctgagaaat catcatgtga 360
   caaacatcat catagcgagt gtgctgggca agggacgcca gcagttggta aatgatcggg 420
   cgacaattcc ttgggtaaca aacaaacgag ttgcagagtg tcaggtgacc ttgcttagtg 480
   tagtagttgg actggataat gaatagcaga gcaccttgaa atctgaggac gttatagttg 540
   ttttaaaaat gtcttcacaa ggccctcatg ttgtgtaaag gagattttaa aattttagtt 600
   caacaaaaag aagacatgtt tactgtgtat cttagcctgt tttagttctg ggacagacac 660
   catagatacc gagctgaaac aacaagtgct ctttacccac agttccaggg cgtgggaagt 720
   acaacatcaa agctctgatg caagtggtgt ccgtgaggat ccatttcctg ctatgcagat 780
   gctgcaaaca tatgactccg gcctcttcct cttctttaag ggcatgtgtc tgaagtgggg 840
   tggggtggca tcctcatgac ttcatctgtg ttttccctag agatagcccc tttggttagg 900
   gttttaacat agatgtttgg tgcacacata catttagtct atgatagtct ctagcaacag 960
   ccagttggga gggaaggaga gggagaggga gagagagaga gagagagaac acatgtatgc 1020
   gagtctgtga tttctagctt cttagcttct tatatttgat ctaattttca tttattttat 1080
   gattctggaa ctctgttgtt ttattacttt gaaattctag gactccaaat aaatattttt 1140
   gatcttaaaa tattatcatt attttattct gatcccagaa ttc                  1183

## Description

La présente invention est relative à des mammifères non-humains transgéniques ou recombinants, dans lesquels l'expression du gène codant pour une protéine associée aux microtubules (MAP) est modifiée (inactivation ou surexpression) et à leurs applications dans le criblage de médicaments utiles dans l'anxiété, la schizophrénie et les désordres schizoaffectifs, à composante anxieuse, paranoïaque ou dépressive.

Les microtubules des cellules de mammifères sont soumis à une régulation : lors de l'interphase, ils organisent l'espace intracellulaire et sont responsables du transport intracellulaire d'organelles ; lors de la mitose, ils se réorganisent pour former le fuseau mitotique, responsable de la répartition des chromosomes entre les deux cellules filles.

Les microtubules, assemblés *in vitro* à partir de solutions de tubuline purifiée, sont labiles et rapidement dépolymérisés par l'exposition au froid.

Des comportements semblables sont observés *in vivo,* mais sont, dans ce cas, régulés par le métabolisme cellulaire. Par exemple, la dépolymérisation des microtubules est favorisée par la phosphoprotéine stathmine, qui lie et séquestre les dimères de tubuline, alors que la stabilisation des microtubules est médiée par les protéines associées aux microtubules (MAPs), qui s'associent aux polymères.

Les neurones contiennent des quantités massives de microtubules et ceux-ci sont en quasi-totalité stables au froid. A partir de préparations de microtubules stables neuronaux, une protéine calmoduline-régulée capable de stabiliser totalement les microtubules (c'est-à-dire apte à supprimer leur activité dynamique et à les rendre résistants au froid) a été isolée ; il s'agit de la protéine STOP (pour *Stable Tubulin Only Polypeptide).* La nature moléculaire de cette protéine est longtemps restée énigmatique. Un pas décisif a été franchi en 1996 lorsque l'ADNc codant pour cette protéine STOP a été cloné (Christophe Bosc et al., P.N.A.S., 1996, **93**, 2125-2130).

La protéine STOP, qui peut bloquer de manière réversible la dynamique des microtubules en abolissant complètement la sensibilité des microtubules neuronaux au froid et aux drogues dépolymérisantes, comporte deux domaines répétés remarquables : un domaine central composé de cinq répétitions quasi-parfaites d'un motif de 46 acides aminés et un domaine carboxy-terminal formé de vingt huit répétitions imparfaites d'un motif de 11 acides aminés. Ces deux domaines répétés sont séparés par une séquence contenant en abondance des résidus lysine et arginine (domaine KR) et par une séquence de liaison ou séquence *linker.* Le domaine N-terminal de la protéine STOP contient des séquences riches en proline qui constituent des sites de liaison potentiels avec des domaines SH3 (*src homology domain 3*).

La structure en exons du gène STOP de souris a été élucidée (Eric Denarier et al., B.B.R.C., 1998, **243**, 791-796). Cette structure correspond à la structure en domaines de la protéine : l'exon 1 code pour le domaine N-terminal y compris le domaine répété central, l'exon 2 code pour la séquence *linker,* l'exon 3 code pour le domaine KR et l'exon 4 code pour la région répétée carboxy-terminale. -

La distribution et le rôle de la protéine STOP dans les neurones ont été récemment caractérisés (Laurent Guillaud et al., *Cell Biol.,* 1998,**142**, 1, 167-179). La distribution de la protéine a été étudiée au niveau ultrastructural dans des cellules neuronales embryonnaires, les cellules DRG (*Dorsal Root Ganglia*), qui peuvent se différencier *in vitro* et a révélé l'existence d'isoformes de cette protéine. Une isoforme majeure (protéine E-STOP) a été caractérisée : cette isoforme apparaît plus tôt dans le développement que la protéine STOP standard ou protéine N-STOP et est majoritaire dans le cerveau embryonnaire. L'ADNc d'E-STOP présente la séquence correspondant au numéro d'accession Genbank AJ002556. La protéine E-STOP diffère de la protéine N-STOP par la délétion des séquences carboxy-terminales répétées, codées par l'exon 4 ; il s'agit donc d'un variant d'épissage de la protéine STOP. Une autre isoforme, la protéine F-STOP a été observée dans des fibroblastes de souris (cellules 3T3). Cette protéine, de masse moléculaire apparente de 45 kDa, est beaucoup plus petite que la protéine N-STOP (115 kDa) ou que la protéine E-STOP (88 kDa). Le clonage et le séquençage de l'ADNc correspondant (Genbank Y16032 et Eric Denarier et al., *P.N.A.S.,* 1998, **95**, 6055-6060) ont montré que les séquences codées par les exons 3 et 4 (KR et répétitions carboxy-terminales) sont absentes dans la protéine F-STOP. De plus, la majeure partie du domaine N-terminal de la protéine N-STOP, située en amont des répétitions centrales et codée par l'exon 1, est absente dans la protéine F-STOP. La protéine F-STOP comprend donc les séquences codées par l'exon 2 et une partie de celles correspondant à l'exon 1, incluant les répétitions centrales. Malgré de multiples délétions, la protéine F-STOP a les mêmes propriétés fonctionnelles de base que la protéine N-STOP : la protéine F-STOP se lie à la calmoduline et a la capacité d'induire une stabilisation au froid des microtubules, *in vitro* et *in vivo.* Contrairement à la protéine N-STOP qui semble presque en permanence associée aux microtubules, la protéine F-STOP reste dans la phase soluble dans les cellules en interphase et ne s'associe aux microtubules que lors de l'exposition au froid. Apparemment, des mécanismes de régulation empêchent la protéine F-STOP d'interagir avec le cytosquelette microtubulaire en interphase, permettant ainsi une dynamique microtubulaire rapide, et ces régulations sont inhibées dès que les cellules sont exposées à des températures basses. Dans les cellules mitotiques, la protéine F-STOP est associée aux fuseaux de microtubules, à température physiologique. -

Ainsi, une seule et même classe de protéines, les protéines STOP, est responsable de la stabilisation des microtubules dans plusieurs types cellulaires différents.

Les formes N, E et F de la protéine STOP ne sont pas les seules isoformes existantes ; en effet, les protéines STOPs sont présentes dans de nombreux tissus, en particulier dans les poumons qui contiennent une isoforme spécifique. De même, la forme F-STOP paraît présente dans des tissus variés. Par contre, les protéines N-STOP et E-STOP sont, semble-t-il, strictement neuronales (C. Bosc et al., *Cell Struct. Function,* 1999, **24**, 393-399).

Il semblerait que la stabilité des microtubules soit importante pour le développement et le maintien de la morphologie et de la fonction des neurones (Laurent Guillaud et al., précité). Ainsi, il a été montré que l'inhibition des protéines *STOP in vitro* par l'injection d'anticorps bloquants spécifiques, supprime la stabilité des microtubules au froid, dans des cellules neuronales ou non-neuronales (Eric Denarier, P.N.A.S., 1998, précité). Il a également été montré que l'inhibition des protéines STOP *in vitro* dans des neurones, altère la différenciation neuronale (Laurent Guillaud et al., précité).

Les Inventeurs ont trouvé, de manière inattendue, que l'invalidation des différentes isoformes de la protéine STOP permet d'obtenir des animaux et notamment des souris particulièrement utiles pour le criblage des médicaments psychoactifs.

La présente invention a en conséquence pour objet un mammifère non-humain recombinant, porteur d'au moins un allèle modifié du gène codant une protéine STOP.

On entend par gène STOP modifié, aussi bien un gène altéré (animaux *knock-in),* qu'un gène inactivé totalement ou partiellement, inhibé ou tronqué (animaux *knock-out).*

De manière avantageuse, lesdits animaux recombinants ou transgéniques sont susceptibles d'être obtenus par recombinaison homologue dans une cellule souche embryonnaire :
- soit par insertion d'au moins un codon STOP ou d'une séquence anti-sens,
- soit par délétion d'une partie ou de tout le gène natif (région codante ou régions non codantes, promoteur, séquences régulatrices en 3', activateurs),
- soit par substitution de séquence.

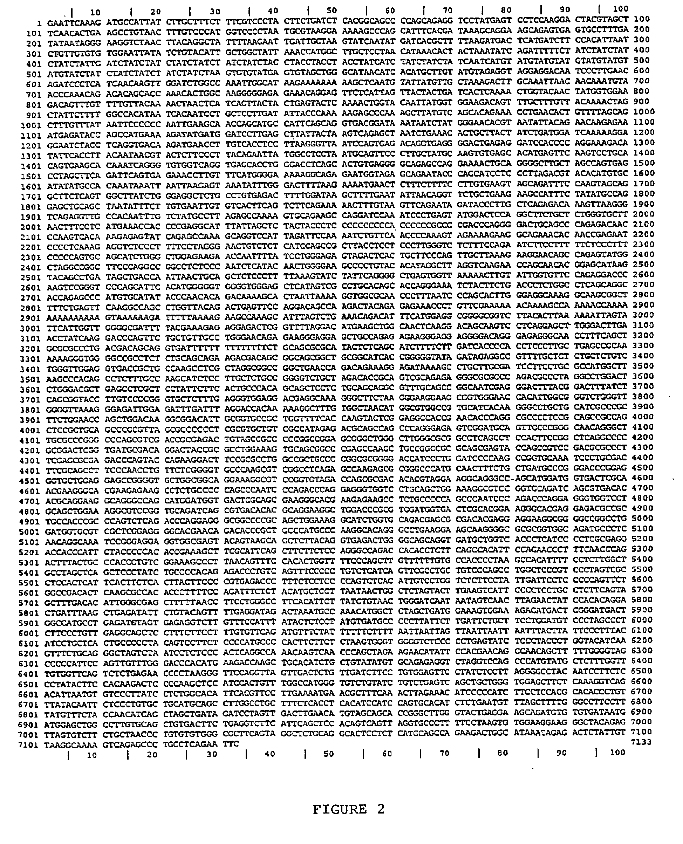

De manière plus précise, une construction conforme à l'invention est avantageusement sélectionnée dans le groupe constitué par :
- des constructions contenant une séquence codant pour une protéine STOP anti-sens, qui va bloquer l'expression de la séquence STOP native,
- des constructions comprenant la région du promoteur STOP (positions 1-3400 de la figure 2) en combinaison avec un gène rapporteur ou avec la région codante STOP. Des marqueurs de sélection positive ou négative peuvent avantageusement être inclus, tel que lacZ, dont la régulation de l'expression entraînera la détection d'un changement du phénotype. Un gène rapporteur préféré est le gène de la GFP (*green fluorescent protein*).
- des constructions comprenant au moins une portion du gène STOP (région codante ou régions non codantes, promoteur, séquences régulatrices en 3', activateurs) incluant la/les modifications souhaitées (délétions, mutations etc..) ; de manière avantageuse, les constructions d'ADN utilisées pour une intégration ciblée doivent inclure une région présentant une homologie avec la séquence cible (gène STOP), pour induire une recombinaison.
- des constructions comprenant au moins une portion du gène STOP, liée de manière opérationnelle à un promoteur, qui peut être constitutif ou inductible et à d'autres séquences régulatrices nécessaires pour l'expression dans l'animal hôte. Par lier de manière opérationnelle, on entend qu'une séquence d'ADN et une séquence régulatrice sont associées de telle manière qu'elles permettent l'expression du gène lorsque les molécules appropriées, par exemple les protéines activatrices transcriptionnelles, sont liées aux séquences régulatrices.

On entend par gène STOP, au sens de la présente invention, les gènes STOP obtenus à partir de n'importe quel mammifère tel que rat, souris, boeuf ou homme, ou du poulet ou du poisson fugu ainsi que les différentes formes mutées dudit gène STOP ; il inclut également les différents cadres ouverts de lecture, les exons, les introns, les régions non-codantes en 3' et en 5' impliquées dans la régulation de l'expression de ce gène, jusqu'à environ 4 kb de part et d'autre de la région codante, le promoteur et les activateurs.

De manière préférée, les constructions sont sélectionnées parmi les constructions suivantes :
- des constructions qui incluent un fragment de la séquence génomique codant pour une protéine STOP comprise entre le codon d'initiation et le codon STOP (C. Bosc et al., E. Denarier et al., L. Guillaud et al. précités), incluant notamment tous les introns normalement présents dans le chromosome natif. Elle peut inclure les régions non traduites en 3' et en 5' retrouvées dans l'ARNm mature. Elle peut, en outre, inclure des séquences de régulation de la transcription ou de la traduction (promoteur, activateur...), incluant environ 4 kb, des régions génomiques flanquantes en 3' ou en 5'.
- des constructions ne comprenant pas la région comprise entre les positions 4118 et 5131 de la séquence génomique codant pour une protéine STOP.
- des constructions comprenant 4,1 kb du gène STOP (correspondant aux positions 1-4118 de la figure 2), le gène codant pour la β-galactosidase, placé sous le contrôle du promoteur STOP endogène, un gène de résistance à la néomycine sous le contrôle du promoteur PGK, 1,57 kb de séquence du gène STOP (correspondant aux positions 5131-6701 de la séquence de la figure 2) et enfin le gène de la thymidine kinase, sous le contrôle du promoteur PGK.

Conformément à l'invention, les animaux transgéniques obtenus constituent deux groupes, les animaux *knock-out* et les animaux *knock-in.*

Dans le cadre de la présente invention :
- les animaux *knock-out* ont une perte de fonction partielle ou complète dans un ou les deux allèles du gène codant pour une protéine STOP endogène ; un tel gène modifié n'induit plus d'expression de la protéine STOP correspondante. Les animaux *knock-out* selon l'invention incluent également des animaux *knock-out* conditionnels : (i) modification du gène codant pour une protéine STOP qui n'intervient qu'après exposition de l'animal à une substance qui induit la modification dudit gène, (ii) introduction d'une enzyme qui induit la recombinaison au niveau d'un site du gène codant pour une protéine STOP (Cre dans le système Cre-lox, par exemple) ou (iii) autre méthode qui induit une modification du gène codant pour une protéine STOP après la naissance.
- les animaux *knock-in* présentent un transgène qui altère le gène endogène codant pour une protéine STOP. Un animal *knock-in* correspond à une altération dans les cellules de l'hôte qui entraîne une expression modifiée ou une fonction modifiée du gène STOP natif. Une expression augmentée ou diminuée peut être ainsi obtenue par introduction d'une copie additionnelle du gène STOP ou par insertion opérationnelle d'une séquence de régulation qui produit une expression significativement augmentée d'une copie endogène du gène STOP. Ces changements peuvent être soit constitutifs, soit conditionnels, en fonction de la présence d'un activateur ou d'un répresseur. Le gène exogène est soit obtenu à partir d'une espèce différente de celle de l'animal hôte ou est modifié au niveau de sa séquence codante ou non codante. Le gène introduit peut être un gène du type sauvage ou une séquence manipulée, par exemple présentant des délétions, des substitutions ou des insertions dans les régions codantes ou non codantes.

Les deux méthodes peuvent être combinées : dans un premier temps le gène d'origine est invalidé, puis dans un deuxième temps, une forme modifiée dudit gène est introduite dans ledit animal.

Les animaux recombinants ou transgéniques ainsi obtenus comprennent une séquence d'acide nucléique exogène, présente, soit sous la forme d'un élément extra-chromosomique, soit intégrée de manière stable dans tout ou une partie des cellules dudit animal, plus particulièrement les cellules germinales.

De manière surprenante, les souris homozygotes contenant les deux allèles du gène STOP inactivés (souris *knock-out* ou STOP KO (-/-)), obtenues par croisement d'animaux hétérozygotes sont viables et ne présentent aucune modification anatomique du cerveau ; en revanche, elles présentent des déficits de la plasticité synaptique, associés à des troubles majeurs multiples du comportement comprenant un défaut complet de maternage, une profonde anxiété, une incapacité à reconnaître les objets et des interactions sociales anormales.

De façon très intéressante, ces troubles multiples du comportement peuvent être améliorés par l'administration prolongée de neuroleptiques.

En conséquence, les souris dans lesquelles le gène STOP a été inactivé (souris STOP KO (-/-)) constituent un modèle particulièrement utile pour l'étude et le traitement des maladies impliquant un défaut synaptique qui sont sensibles aux neuroleptiques, notamment la schizophrénie et les désordres schizoaffectifs à composante anxieuse, paranoïaque ou dépressive.

La présente invention a également pour objet l'utilisation dudit mammifère non-humain recombinant porteur d'au moins un allèle du gène codant pour une protéine STOP modifié, pour la sélection ou le criblage de produits psychoactifs.

Les molécules d'acide nucléique comprenant la séquence d'un allèle modifié du gène codant pour une protéine STOP tel que défini ci-dessus (notamment les séquences de gènes STOP inactivés) sont notamment obtenues par mutation, de différentes manières connues en elles-mêmes, pour générer les modifications ciblées recherchées : substitutions, insertions ou délétions au niveau d'un domaine ou d'un exon, qui conduisent à l'expression d'une protéine STOP inactivée ou à l'absence d'expression de protéine STOP. Les délétions peuvent inclure des modifications importantes : délétion d'un domaine ou d'un exon (exon 1 notamment).

Les fragments desdites séquences sont avantageusement obtenus par synthèse chimique d'oligonucléotides, par digestion enzymatique ou par amplification PCR par exemple.

Lesdits fragments comprennent au moins 15 nucléotides, de préférence environ 18 nucléotides et de manière préférée au moins 50 nucléotides.

De tels fragments sont utiles comme amorces de PCR ou pour le criblage par hybridation, des cellules ES recombinantes ou des animaux non-humains recombinants.

Lesdites amorces ou sondes de criblage de clones ES recombinants ou d'animaux recombinants sont caractérisées en ce qu'elles sont sélectionnées dans le groupe constitué par des fragments d'un gène STOP comprenant au moins 15 nucléotides, de préférence environ 18 nucléotides et de manière préférée au moins 50 nucléotides. De telles amorces ou sondes permettent de cribler des cellules ou des animaux comprenant l'une des séquences modifiées, telles que définies ci-dessus.

De manière préférée, les amorces suivantes sont utilisées pour le criblage :
. oligonucléotide A4080 : positions 4067-4095 de la figure 2 (SEQ ID NO:3) ;
. oligonucléotide 770 : positions 4488-4515 de la figure 2 (SEQ ID NO:4) ;
. oligonucléotide AS2 : positions 6680-6701 de la figure 2 (SEQ ID NO:5).

Des fragments plus importants (plus de 100 nucléotides) sont utiles pour la production des protéines STOP.

Les séquences homologues aux séquences STOP clonées sont identifiées par différentes méthodes connues de l'Homme du métier.

La similarité des séquences d'acides nucléiques est détectée par hybridation dans des conditions de faible stringence, par exemple à 50°C et 10 x SSC (0,9 M de tampon salin et 0,09 M de citrate de sodium).

Lesdites séquences restent associées lorsqu'elles sont soumises à un lavage à 55°C dans un tampon 1 x SSC.

L'identité des séquences peut être déterminée par hybridation dans des conditions stringentes, par exemple à 50°C au plus et 0,1 x SSC (9 mM de tampon salin/0,9 mM de citrate de sodium).

La présente invention a également pour objet des sondes de détection et de criblage de l'ADN génomique par hybridation des clones ES recombinants ou des animaux recombinants, caractérisées en ce qu'elles sont constituées par un fragment du même gène STOP, situé en dehors (amont ou aval) de la séquence du gène STOP issue du vecteur de recombinaison mis en oeuvre (zone de recombinaison homologue).

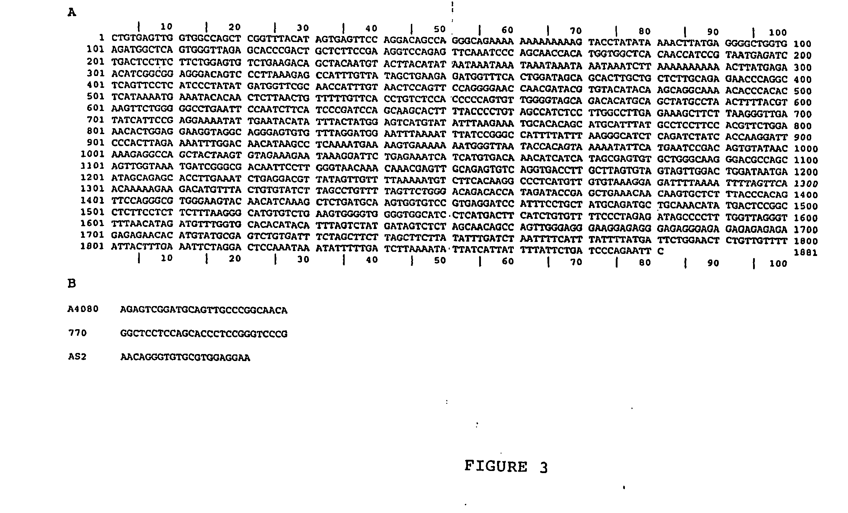

De manière avantageuse, ladite sonde correspond aux positions 700-1881 de la figure 3 (SEQ ID NO:6).

La présente invention a également pour objet un procédé de criblage et de sélection de molécules utiles dans le traitement de la schizophrénie et des désordres schizoaffectifs à composante anxieuse, paranoïaque ou dépressive, caractérisé en ce qu'il comprend au moins les étapes suivantes :
- la mise en contact *in vitro* d'au moins une substance à cribler avec un échantillon biologique constitué par un extrait de cellules ou des tranches d'organes, de préférence neuronales ou cérébrales, obtenu à partir d'au moins un mammifère non-humain recombinant porteur d'au moins un allèle modifié du gène codant pour une protéine STOP,
- la mesure de l'action de ladite substance à cribler sur lesdites cellules ou tranches d'organes et
- la comparaison des valeurs obtenues avec celles des cellules ou des tranches d'organes d'un échantillon biologique obtenu à partir d'un mammifère non-humain du même type, porteur de deux allèles sauvages du gène codant pour une protéine STOP.

Les substances testées sont notamment obtenues à partir de banques de substances (naturelles ou synthétiques).

Selon un mode de mise en oeuvre avantageux dudit procédé, ladite mesure est effectuée à l'aide d'un test de liaison protéine-protéine ; dans un tel cas, une ou plusieurs des molécules mises en oeuvre peuvent être marquées par un marqueur ; ledit marqueur peut fournir un signal détectable soit directement, soit indirectement.

Parmi les marqueurs utilisables, on peut citer par exemple, les radioisotopes, les molécules fluorescentes, chemiluminescentes, les enzymes, les molécules de liaison spécifique, des particules telles que des particules magnétiques, etc...

Des molécules de liaison spécifique incluent des paires de molécules telles que la biotine et la streptavidine, la digoxine et l'anti-digoxine, etc...

Pour les membres de liaison spécifique, le membre complémentaire sera marqué avec une molécule adaptée à la détection, conformément aux méthodes connues.

Beaucoup d'autres réactifs peuvent être utilisés dans un tel test de criblage ; il inclut par exemple des sels, des protéines neutres telles que l'albumine, des détergents, etc..., qui sont utilisés pour faciliter une liaison protéine-protéine optimale et/ou réduire les interactions non-spécifiques ou de bruit de fond.

Les réactifs qui améliorent l'efficacité de l'essai, tels que les inhibiteurs de protéases, les inhibiteurs de nucléases, les agents anti-microbiens peuvent également être utilisés.

Le mélange de composants est ajouté dans n'importe quel ordre, de manière à permettre la liaison recherchée.

Les incubations sont réalisées à température convenable, usuellement entre 4°C et 40°C.

Les périodes d'incubation peuvent varier ; elles sont habituellement comprises entre 0,1 et 1 h et sont optimisées dans cette gamme de temps, notamment pour faciliter un criblage rapide.

Les anticorps spécifiques des polymorphismes des protéines STOP peuvent être utilisés dans des immunoessais de criblage, plus particulièrement pour détecter la liaison de substrat ou de protéine STOP ou pour confirmer l'absence ou la présence d'une protéine STOP dans une cellule ou un échantillon, tel qu'un échantillon biologique.

Selon un autre mode de mise en oeuvre avantageux dudit procédé, ladite mesure est effectuée par détection de la variation d'intensité d'un signal électrique ; en effet, on peut observer en ce qui concerne les cellules nerveuses des animaux recombinants selon l'invention, une altération de l'organisation de la synapse (positionnement et transport de neurorécepteurs) chez les animaux recombinants selon l'invention.

La présente invention est également relative à un procédé de criblage et de sélection de molécules utiles dans le traitement de l'anxiété, la schizophrénie et des désordres schizoaffectifs à composante anxieuse, paranoïaque ou dépressive, lequel procédé est caractérisé en ce qu'il comprend :
- l'administration à au moins un mammifère non-humain recombinant porteur d'au moins un allèle modifié du gène codant une protéine STOP d'au moins une substance à cribler ; et
- l'étude comportementale dudit mammifère par rapport à une série d'animaux témoins et/ou la détermination de la localisation des médicaments après leur administration.

De tels animaux peuvent avantageusement servir de modèles pour le criblage de molécules psychoactives, présentant une faible toxicité chez l'homme.

La présente invention a également pour objet un vecteur de recombinaison homologue d'un gène codant pour une protéine STOP, caractérisé en ce qu'il comprend une séquence nucléotidique d'un gène STOP modifié codant pour une protéine STOP inactivée, dont un fragment de 1012 pb contenant les séquences répétées de la région codante de l'exon 1 a été délétée et remplacée par une cassette d'expression contenant le gène de résistance à la néomycine (*néo*) sous le contrôle du promoteur PGK et le gène de la β-galactosidase (*lacZ*) sous le contrôle transcriptionnel du promoteur STOP endogène.

La présente invention a également pour objet une méthode de production de mammifères non-humains recombinants porteurs d'au moins un allèle du gène codant pour une protéine STOP inactivée, caractérisée en ce que :
- l'on tronque un allèle du gène codant pour une protéine STOP ;
- l'on introduit ladite séquence modifiée dans un segment de l'ADN génomique d'un mammifère non-humain du même type, associé à un marqueur approprié, de manière à obtenir une séquence marquée M, contenant ledit allèle modifié ;
- l'on intègre ladite séquence M, *in vitro,* dans les cellules souches de lignées germinales d'embryons d'un mammifère non-humain par transfection et on sélectionne les cellules ayant ledit allèle par des évènements de recombinaison homologue ; puis
- l'on réinjecte lesdites cellules souches sélectionnées à un embryon réimplanté chez un mammifère non-humain du même type afin d'obtenir des animaux chimériques ; et
- l'on obtient à la génération F1 des mammifères non-humains recombinés hétérozygotes et à la génération F2 des mammifères non-humains recombinés homozygotes STOP -/-, reconnaissables à la présence du marqueur et des souris dites sauvages (+/+).

Outre les dispositions qui précèdent, l'invention comprend encore d'autres dispositions, qui ressortiront de la description qui va suivre, qui se réfère à des exemples de mise en oeuvre du procédé objet de la présente invention, avec référence aux dessins annexés dans lesquels :

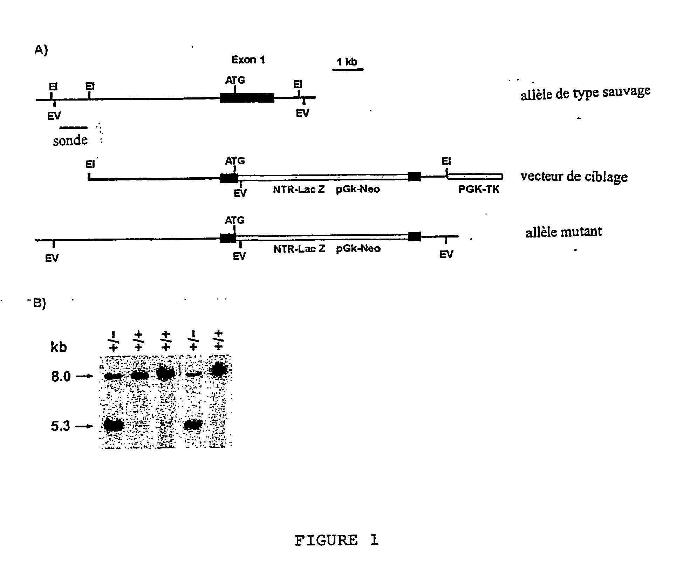

- la figure 1 illustre l'organisation génomique du gène STOP au niveau de l'exon 1 et l'établissement de souris *knock-out* pour le gène STOP [souris STOP KO (-/-)] par altération de l'exon 1. A : carte de restriction d'un fragment du gène STOP (allèle sauvage) utilisé pour la production d'un fragment génomique d'homologie, structure du vecteur de recombinaison homologue ou vecteur de ciblage ptSTOP, et structure prédite de l'allèle mutant. EV : EcoRV ; EI : EcoRI ; TK : thymidine kinase ; pgk : phosphoglycérate kinase ; néo : néomycine ; *NTR : Nucleotide Translation Region.* **B :** profils Southern blot du gène STOP chez des souris sauvages (+/+ ; 8 kb) et hétérozygotes (+/- ; 5,3 kb),
- la figure 2 représente la séquence du gène STOP au niveau de l'exon 1 (positions 3333-5150) (clone génomique de 7,2 kb) ; les fragments utilisés pour l'établissement des souris STOP KO (-/-), sont les suivants : séquence homologue en 5' : 4,118 kb, positions 1-4118 ; séquence homologue en 3' : 1,57 kb, positions 5131 à 6701.
- figure 3 : la figure 3A représente la séquence génomique du gène STOP, située en 5' par rapport à la séquence de la figure 2, et la figure 3B représente la sonde utilisée pour le criblage, qui est un fragment EcoR V-EcoR I (positions 701-1881 sur la figure 3A).

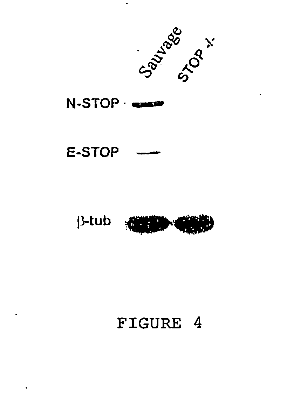

- la figure 4 illustre l'analyse en *Western-blot,* à l'aide de l'anticorps polyclonal 23C (Laurent Guillaud et al., précité), de l'expression des protéines STOP (E-STOP et N-STOP) dans le cerveau des souris STOP KO (-/-) ou sauvages. Cette figure montre l'absence de protéines STOP dans le cerveau des souris STOP KO (-/-), par comparaison avec les souris de type sauvage ; la présence d'une quantité équivalente de protéines dans les deux types d'échantillons déposés sur le gel est démontrée par le signal obtenu avec un anticorps anti-tubuline β (β-tub).

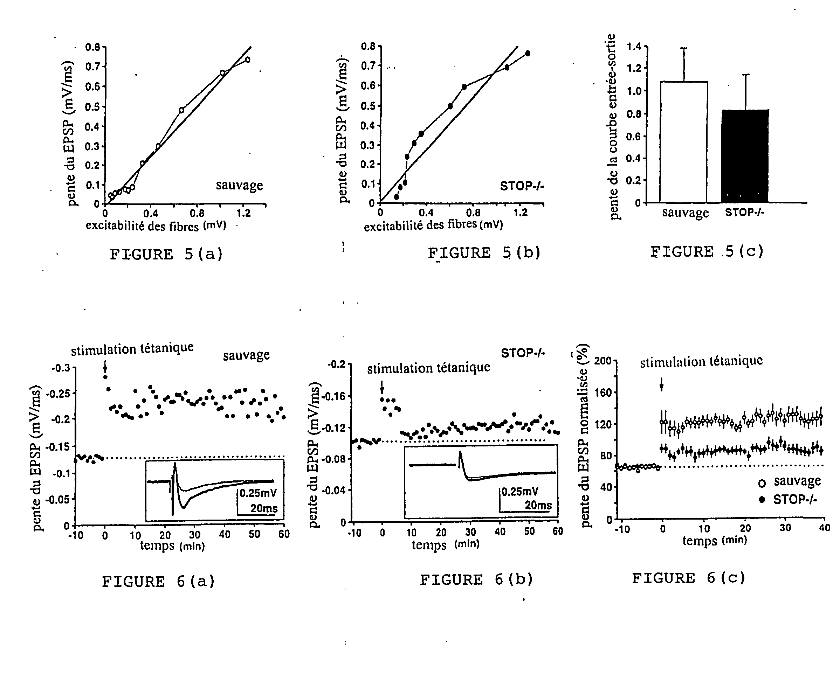

- les figures 5 à 8 illustrent l'altération de la dépression à long terme (LTD pour *long-term depression*) et de la potentialisation à long terme (LTP pour *long-term potentiation*) chez les souris STOP KO (-/-) :

- la figure 5 illustre la réponse synaptique basale des collatérales de Schaffer : les courbes du type "entrée-sortie" représentent la pente de la courbe du potentiel d'excitation post-synaptique (EPSP pour *excitatory post-synaptic potential*) en fonction de l'excitabilité des fibres des collatérales de Schaffer, à partir d'ûne coupe de souris de type sauvage (a) ou de souris STOP KO (-/-) (b). Résumé des résultats obtenus sur six souris de type sauvage et six souris STOP KO (-/-) (c). Les pentes des courbes ne sont pas significativement différentes, indiquant une transmission synaptique basale normale chez les souris STOP KO (-/-),
- la figure 6 illustre les résultats des expériences de potentialisation à long terme (LTP pour *"Long-term potentiation"*) au niveau des synapses des collatérales de Schaffer et des cellules pyramidales de la région CA1 de l'hippocampe :
   - la figure 6(a) montre qu'une stimulation à une fréquence élevée (stimulation tétanique induite par 4 stimuli de 100 Hz pendant 1 s, appliqués à des intervalles de 10 à 20 s) entraîne une augmentation à long terme de la pente de la courbe du EPSP, dans une coupe de souris de type sauvage,
   - la figure 6(b) montre qu'en revanche, une stimulation identique entraîne uniquement une faible augmentation de la pente de la courbe du EPSP dans une coupe de souris STOP KO (-/-), et
   - la figure 6(c) représente un résumé des résultats obtenus chez les souris de type sauvage et les souris STOP KO (-/-). Les valeurs initiales des pentes des courbes du EPSP ont été normalisées dans chaque expérience, en utilisant la valeur moyenne de la courbe obtenue pendant la période témoin (-10 à 0 min). Les résultats exprimés en moyenne ± s.e.m., correspondent aux valeurs obtenues sur 13 et 9 coupes issues respectivement de sept souris de type sauvage et six souris STOP -/-. Ces résultats montrent un déficit significatif de la potentialisation à long terme chez les souris STOP KO (-/-) (p = 0,0007 mesure enregistrée au bout de 30 à 40 minutes),

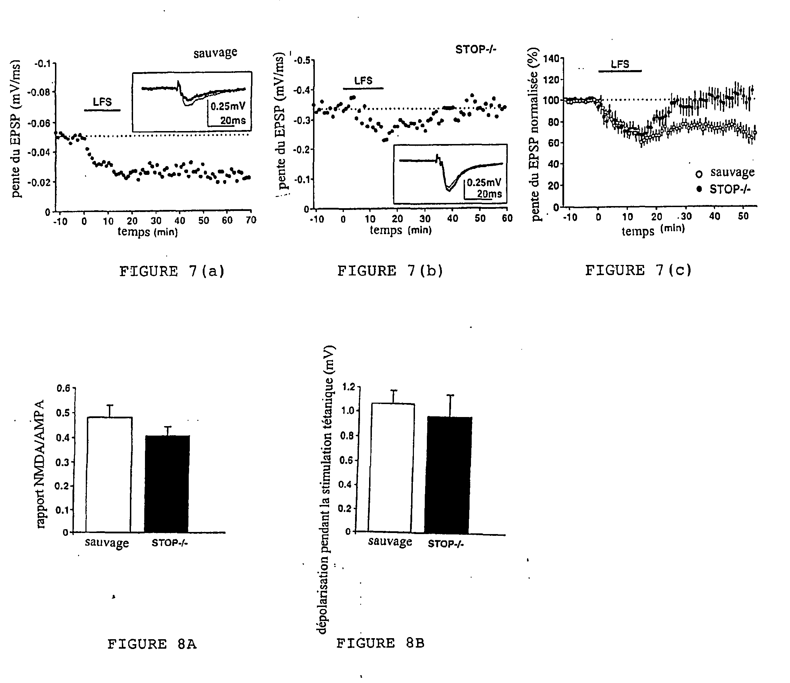

- la figure 7 illustre les résultats des expériences de dépression à long terme (LTD) au niveau des synapses des collatérales de Schaffer et des cellules pyramidales CA1 :
   - la figure 7(a) montre que la stimulation à basse fréquence (LFS pour *low frequency stimulation,* 1Hz pendant 15 min) induit une diminution à long terme de la pente de la courbe du EPSP dans les coupes de souris de type sauvage,
   - la figure 7(b) montre qu'en revanche, la stimulation à basse fréquence n'induit pas de diminution à long terme de la pente de la courbe du EPSP dans les coupes de souris KO -/-, et
   - la figure 7(c) représente un résumé des expériences de LTD dans les souris STOP KO (-/-) et dans les souris de type sauvage. Les résultats exprimés en moyenne +/s.e.m., correspondent aux valeurs obtenues sur 15 et 9 coupes de respectivement neuf souris de type sauvage et six souris STOP KO (-/-). Ces résultats montrent une altération significative de la dépression à long terme (LTD) dans les souris STOP KO (-/-) (p=0,01, résultats enregistrés au bout de 40-45 minutes).
- la figure 8A illustre le rapport NMDA/AMPA au niveau des synapses des collatérales de Schaffer et des cellules pyramidales CA1 correspondant au rapport des valeurs des courbes du EPSP du récepteur du NMDA (N-méthyl-D-aspartate) et du récepteur de l'AMPA (alpha-amino-3-hydroxy-5-méthyl-4-isoxazole acide propionique) sur 14 et 9 coupes de respectivement, six souris de type sauvage et six souris STOP KO (-/-). Les pentes des récepteurs du NMDA et de l'AMPA ont été mesurées pour une intensité de stimulus correspondant à 2 fois la valeur seuil. Aucune différence significative n'a été observée entre les souris de type sauvage et les souris STOP KO (-/-).
- la figure 8B illustre la dépolarisation pendant une stimulation tétanique des collatérales de Schaffer : le graphique représente le résumé des résultats de quantification de la dépolarisation pendant une stimulation tétanique. La dépolarisation est calculée 300 ms après le début du premier stimulus de 100 Hz. Les expériences ont été réalisées sur 11 coupes de souris de type sauvage et 8 coupes de souris STOP KO (-/-) provenant de respectivement sept souris de type sauvage et six souris STOP KO (-/-). Les résultats ne sont pas significativement différents chez les souris de type sauvage et les souris STOP KO (-/-).

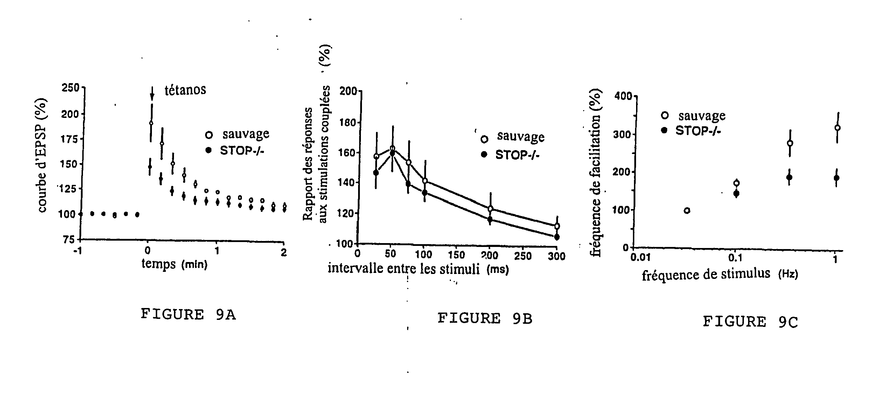

- la figure 9 illustre l'altération de la plasticité synaptique à court terme chez les souris STOP KO (-/-) :
   - la figure 9A illustre les résultats des expériences de potentialisation post-tétanique de la transmission synaptique des collatérales de Schaffer. Une stimulation à haute fréquence en présence de l'antagoniste du récepteur NMDA, D-APV (50-100 µM) induit une augmentation transitoire de la pente du EPSP. Les résultats ont été obtenus à partir de 6 et 10 coupes provenant de respectivement quatre souris de type sauvage et cinq souris STOP KO (-/-). Les résultats montrent une altération de la potentialisation post-tétanique chez les souris STOP KO (-/-) (p=0,04, mesures effectuées de 0 à 30 s après la stimulation tétanique),
   - la figure 9B illustre les résultats des expériences de facilitation par stimulations couplées (PPF for *paired pulse facilitation*), de la transmission synaptique des collatérales de Schaffer. Les résultats obtenus correspondent à 7 et 12 coupes provenant de respectivement quatre souris de type sauvage et cinq souris STOP KO (-/-). La facilitation par stimulations couplées n'est pas modifiée de façon significative chez les souris STOP-/-, et
   - la figure 9C illustre les expériences de facilitation de fréquence des fibres moussues de l'hippocampe. Les résultats ont été obtenus à partir de 10 et 12 coupes obtenues à partir de 7 et 8 coupes provenant de respectivement six souris de type sauvage et sept souris STOP KO (-/-). Dans les souris de type sauvage, une stimulation répétée des synapses de la fibre moussue en utilisant des fréquences de stimulation comprises entre 0,033 et 1 Hz, a provoqué une augmentation réversible, d'un facteur 3, de l'amplitude de la réponse de la fibre moussue. La facilitation est altérée de façon significative chez les souris STOP KO (-/-) (p=0,03, valeurs enregistrées à une fréquence de stimulation de 1 Hz).

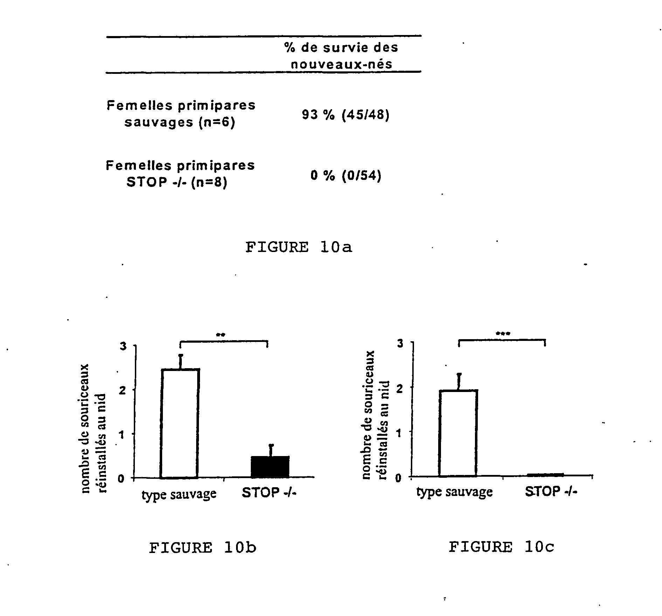

- la figure 10 illustre les troubles du comportement maternel des souris STOP KO (-/-) :
   - figure 10a : la survie des nouveaux-nés, issus de mères primipares, portant l'allèle STOP sauvage ou muté (STOP -/-), est analysée au deuxième jour après la naissance,
   - figures 10b et 10c : la manifestation d'un comportement maternel est analysée chez les jeunes femelles primipares et les jeunes mâles STOP KO (-/-), par comparaison avec les souris de type sauvages. Les résultats sont exprimés sous forme de moyenne ± s.e.m ; n = 9 pour les souris femelle de type sauvage et STOP KO (-/-) et n = 10 pour les souris mâle de type sauvage et STOP KO (-/-).

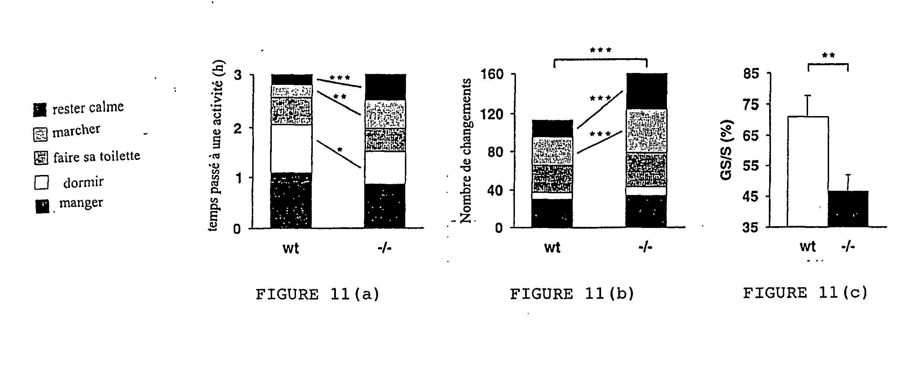

- la figure 11 illustre les troubles du comportement chez les souris STO KO (-/-) ; les activités des souris (dormir, manger, faire sa toilette, marcher et rester immobile tout en étant éveillé) ont été enregistrées en vidéo pendant une durée de 3 heures, n = 11 pour les souris de type sauvage (wt pour *wild-type*) et les souris STOP KO (-/-) :
   - figure 11a : temps consacré à chaque activité. Chaque boîte correspond à une activité différente, comme indiquée dans le panneau de gauche. Les souris STOP KO (-/-) passent plus de temps à marcher et à rester immobiles que les souris de type sauvage, au détriment du temps passé à dormir ou à manger,
   - figure 11b : nombre de changements d'activité. Par rapport aux souris de type sauvage, les souris STOP KO (-/-) montrent un nombre plus élevé de changements d'activités, avec un nombre plus élevé de phases de marche et de repos,
   - figure 11c : pourcentage des phases de toilette suivies d'une phase de sommeil (GS) sur nombre total de phases de sommeil (S) exprimées en moyenne ± s.e.m. Les pourcentages sont calculés pour chaque souris avant de calculer la moyenne. La séquence G-S typique chez les souris de type sauvage est fréquemment interrompue chez les souris STOP KO (-/-).

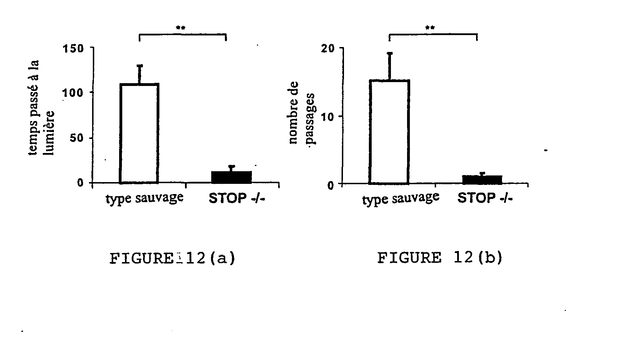

- la figure 12 illustre l'état d'anxiété des souris STOP KO (-/-), évalué par le test de lumière et d'obscurité. figure 12a : le temps passé dans la boîte éclairée et figure 12b : le nombre de passages entre les deux boîtes sont respectivement enregistrés sur une durée de 5 minutes, à partir de la première entrée des animaux dans la boîte obscurcie. Les souris sauvages (+/+) sont utilisées comme témoins. Les valeurs correspondent à valeur moyenne ± erreur standard à la moyenne (s.e.m). Les différences entre les souris KO et les souris témoins sont indiquées avec un risque p < 0,01 (**).

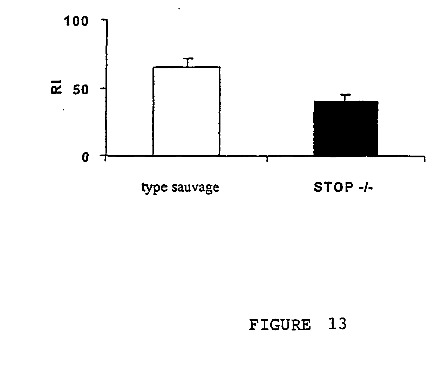

- la figure 13 illustre les troubles de la mémoire à court terme des souris STOP KO (-/-), évalués par le test de reconnaissance d'un objet. Les souris sauvages (+/+) sont utilisées comme témoins. Les résultats sont exprimés par l'indice de reconnaissance (RI) : les valeurs de RI significativement supérieures à 50% correspondent à un test de reconnaissance positif. Les valeurs correspondent à la valeur moyenne ± erreur standard à la moyenne (s.e.m).

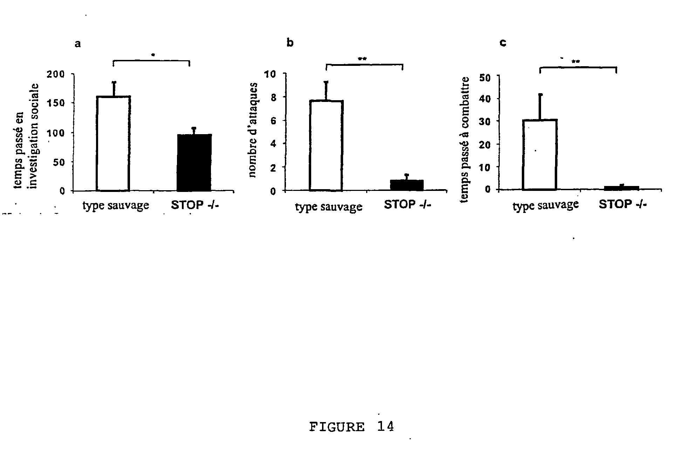

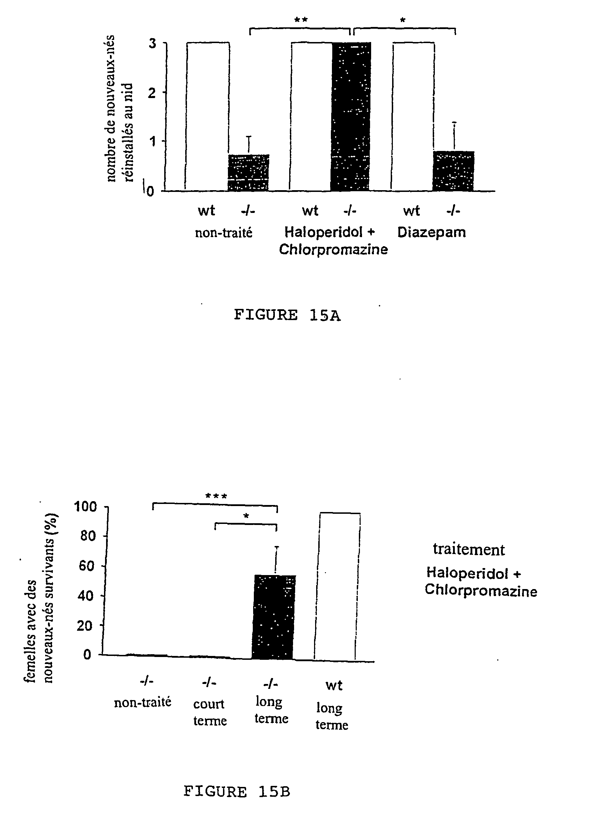

- la figure 14 illustre le comportement social des souris STOP KO (-/-) ; les souris sauvages (+/+) sont utilisées comme témoins : figure 15a : évaluation du temps passé par un mâle à l'investigation sociale, vis-à-vis d'un intrus [n=11 pour les souris sauvages et n=13 pour les souris STOP KO (-/-)] ; figure 15b : agression inter-mâle ; les tests d'agression sont réalisés pendant deux jours consécutifs [n=11 pour les souris sauvages et n=10 pour les souris STOP KO (-/-)] ; le nombre d'agressions et le temps passé à combattre (moyenne ± erreur standard à la moyenne (s.e.m)) sont enregistrés le deuxième jour; * : p<0,05, ** : p<0,01, test U Mann-Whitney.
- la figure 15 illustre l'effet des neuroleptiques sur le comportement maternel chez les souris STOP KO (-/-) :
   - figure 15A : Réinstallation au nid des nouveaux-nés issus de femelles post-partum de type sauvage (wt) et STOP KO (-/-). La réinstallation des souriceaux au nid a été testée pendant le premier jour post-partum, chez les souris traitées avec des neuroleptiques (mélange d'halopéridol et de chlorpromazine), un anxiolytique (diazépam) ou non-traitées. Les souris ont reçu une dose de 0,5 mg/kg/jour de 6-8 jours avant l'accouchement jusqu'au jour de l'accouchement. Les femelles ont été mises en présence de 3 nouveaux-nés et la réinstallation au nid a été enregistrée pour chaque femelle. La moyenne des valeurs obtenues est présentée pour chaque génotype (moyenne ± s.e.m., n = 6 pour chaque groupe de souris de type sauvage et STOP KO (-/-). * p < 0,05, ** p< 0,02, *** p<0,01, Test U de Mann et Whitney non paramétrique.
   - figure 15B : Survie des nouveaux-nés chez les souris de type sauvage et les souris STOP KO (-/-). La survie des nouveaux-nés est analysée chez les souris STOP KO (-/-) soumises à différents traitements. Les nouveaux-nés sont considérés comme survivants lorsqu'ils sont élevés jusqu'au sevrage. Aucune survie des nouveaux-nés n'a été observée chez les souris STOP KO (-/-) non-traitées (n = 20) ou traitées à court terme (figure 15A). En revanche, une survie des nouveaux-nés est observée chez quatre des sept souris STOP KO (-/-) traitées à long terme (4 mois) avec des neuroleptiques. Une survie des nouveaux-nés est observée chez toutes les souris de type sauvage (n = 7) soumises au même traitement à long terme avec des neuroleptiques. *p < 0,05, **p< 0,02, ***p<0,01, test exact de Fisher.

### Exemple 1: Établissement de souris "Knock out" (KO) dans lesquelles le gène STOP est inactivé : souris STOP KO (-/-)

### 1- Matériels et méthodes

### 1-1 Construction du fragment, génomique d'homologie et du vecteur de recombinaison homologue (vecteur de ciblage).

Les fragments d'ADN génomique, utilisés pour construire le vecteur de recombinaison homologue sont issus d'une banque d'ADN génomique de souris de souche 129 clonée dans le phage P1 et criblée par hybridation avec un ADNc du gène STOP ou une sonde d'ADNc dudit gène (Eric Denarier et al., B.B.R.C.,1998, précité).

Le fragment génomique d'homologie du gène STOP est construit à partir du clone de 7,2 kb dont la séquence est présentée à la figure 2, selon les étapes suivantes : un fragment de 1012 pb, contenant les séquences répétées de la région codante du gène STOP qui s'étend des positions 4118 à 5131 de la séquence présentée à la figure 2, a été délété et remplacé par une cassette d'expression contenant le gène de résistance à la néomycine (*néo*) sous le contrôle du promoteur PGK et le gène de la β-galactosidase (*lacZ*) sous le contrôle transcriptionnel du promoteur STOP endogène. En outre, un site EcoRV a été introduit en 5' du gène *lacZ.*

Le vecteur de recombinaison homologue (ptSTOP) est obtenu par clonage du fragment d'homologie du gène STOP précédemment décrit dans le vecteur pGK-TK. Le vecteur pGK-TK dérive du vecteur pPNT construit par Tybulewicz et al. (Cell, 1991, 65, 1153-1163) par insertion du gène de la thymidine kinase du virus de l'Herpès simplex (HSV) sous le contrôle du promoteur de la phosphoglycérate kinase (PGK).

### 1-2 Recombinaison homologue dans les cellules ES et génotypage

Le vecteur ptSTOP est linéarisé par l'enzyme Not1 et électroporé dans des cellules ES (ES-R1, A. Naguy et al., P.N.A.S., 1993, **90**, 8428-8428) ou dans les cellules ES-AT1 isolées à partir de blastocystes de 3,5 jours issus de souris F1 (129 Sv Pas x 129 Sv Pas). Ensuite, les cellules ES électroporées sont ensemencées sur une couche de fibroblastes résistants à la néomycine, préalablement traités à la mitomycine, et cultivées dans du milieu DMEM riche en glucose (INVITROGEN) contenant 15 % de sérum de veau foetal et 1000 UI/ml de facteur inhibiteur de leucémie (Esgron, CHEMICON). 2 jours après la transfection, de la généticine (G418, INVITROGEN) est ajoutée dans le milieu de culture, à la concentration finale de 250 µg/ml. Le gancyclovir (SYNTEX) est ajouté du quatrième au huitième jour après la transfection. Les clones de cellules ES recombinantes sont prélevées 10 jours après la transfection et amplifiées avant d'être congelées ou analysées. Le génotype des clones résistant au G418 et au gancyclovir est vérifié par analyse en Southem blot de l'ADN génomique digéré par EcoRV et hybridé avec une sonde spécifique du gène STOP, située dans la région 5' flanquant la région de recombinaison homologue (voir figure 3) et correspondant aux positions 698-1875, après digestion EcoRV-EcoRI de la figure 3. La taille des fragments de restriction est de 8 kb pour l'allèle sauvage et de 5,3 kb pour l'allèle muté (Figure 1B).

### 1-3 Microinjection des cellules ES recombinantes et obtention de souris transgéniques homozygotes pour l'allèle muté du gène STOP [souris STOP KO (-/-))].

Les cellules ES recombinantes portant l'allèle muté sont microinjectées dans des embryons de souris OF1 au stade morula, puis les embryons injectés sont réimplantés dans l'utérus de la mère porteuse pour donner naissance à des souris chimères (*Gene targeting : a practical approach, A. L. Joyner Ed., New York, Oxford University Press, 1993, pages 174-179*). Le croisement de ces chimères avec des souris BalB/c ou 129/sv (Laboratoires CHARLES RIVER) produit des descendants F1 hétérozygotes, dans lesquels la transmission de la mutation du gène STOP est vérifiée par analyse en Southem blot de l'ADN génomique provenant d'un échantillon de queue. Les descendants F1 sont croisés entre eux pour donner des descendants F2 homozygotes.

### 1-4 Analyse en Western-blot de l'expression du gène STOP dans le cerveau des souris STOP KO (-/-)

Des extraits de cerveaux de souris STOP KO (-/-) et de souris de type sauvage sont préparés et analysés en Western-blot à l'aide de l'anticorps polyclonal 23C, selon les protocoles décrits dans Guillaud et al. , précité.

### 1-5 Analyse histologique, immunomarquage des protéines STOP et détection de l'activité β-galactosidase du cerveau des souris STOP KO (-/-)

### a) analyse histologique

Des souris mâle sauvages et STOP KO (-/-) âgées de 10 à 12 semaines sont perfusées avec une solution de paraformaldéhyde (PFA 4 %). Les cerveaux sont fixés dans la même solution, pendant 2h à 4°C. Un test de détection de la cytochrome oxydase (Y. Liu et al., J. Neurosci. Methods, 1993, 49, 181-184) et une coloration au cristal violet sont effectués sur des coupes de 100 µm des cerveaux.

### b) immunomarquage des protéines STOP

Les cerveaux sont préparés comme décrit au paragraphe a) puis congelés dans du saccharose (20% dans PBS). Des coupes de cerveau de 20 µm sont incubées successivement dans les solutions suivantes : 1 % H₂O₂ (15 min), 3 % de BSA (30 min) et mélange de l'anticorps polyclonal 23C (100 µg/ml) et du conjugué anti-anticorps de lapin couplé à la peroxydase (une nuit), puis les protéines STOP sont révélées par l'éthylcarbazole (AEC, DAKO).

### c) détection de l'activité β-galactosidase

Des coupes de cerveaux (100 µm) sont fixées dans 0,2 % de glutaraldéhyde et 2% de formaldéhyde. L'activité β-galactosidase est détectée par coloration des coupes dans une solution de PBS contenant 5 mM de ferricyanure de potassium, 5 mM de ferrocyanure de potassium, 2 mM de chlorure de magnésium et 1 mg/ml de X-Gal, à 30°C, pendant 3 à 5 heures.

### 1-6 Analyse de la stabilité des microtubules dans les neurones et les cellules gliales des souris STOP KO (-/-)

Des neurones et des cellules gliales d'embryons de souris de type sauvage et de souris STOP KO (-/-) sont maintenues à température ambiante ou soumises à une tempéraure de 0° C pendant 45 minutes. Après extraction de la tubuline libre, selon le protocole décrit dans Laurent Guillaud et al., précité, les microtubules sont colorés avec un anticorps anti-tubuline et les noyaux sont colorés par la solution de Hoechst.

### 2- Résultats

### 2-1 Etablissement de souris STOP KO (-/-)

Le profil génotypique du mutant homozygote STOP KO (-/-) montre la présence d'un fragment de 5,3 kb (figure 1B) qui indique la délétion du fragment de 1012 pb contenant les séquences répétées de la région codante du gène STOP.

L'analyse des différents croisements hétérozygotes montre que l'allèle STOP muté est transmis de manière mendélienne.

Les souris homozygotes STOP KO (-/-) sont viables, apparaissent en bonne santé et ne présentent pas de lésions macroscopiques visibles.

On obtient un phénotype nul ; les souris portant l'allèle muté à l'état homozygote n'expriment pas de protéine STOP :
- l'analyse des extraits de cerveaux des souris STOP KO (-/-), en Western-blot à l'aide de l'anticorps polyclonal 23C (Guillaud et al., précité), montre une absence de protéines STOP (E-STOP et N-STOP) chez ces souris alors que ces deux isoformes sont détectées chez les souris adultes de type sauvage (figure 4).
- l'analyse immunohistologique des coupes de cerveaux des souris STOP KO (-/-) montre une absence de marquage spécifique des protéines STOP (E-STOP et N-STOP) chez ces souris alors qu'un marquage spécifique de ces protéines STOP est observé dans l'ensemble des tissus nerveux des souris adultes de type sauvage.

### 2-2 Absence de stabilité au froid des microtubules de cellules issues de souris STOP KO (-/-)

Du fait de l'absence de protéine STOP chez les souris STOP KO (-/-) , on observe une dépolymérisation des microtubules après exposition au froid, à la fois dans les neurones, les cellules gliales ou les fibroblastes.

### 2-3 Absence de lésions anatomiques dans le cerveau des souris STOP KO (-/-)

L'analyse en microscopie optique, de l'anatomie du cerveau des souris STOP KO (-/-), à partir de coupes parasagitales colorées au cristal violet pour visualiser les noyaux, ne montre pas de différences entre les souris KO -/- et les souris de type sauvage.

Plus précisément :
- l'analyse des couches de cellules du cervelet, du néocortex, de l'hippocampe et du bulbe olfactif qui correspondent à celles montrant une expression importante des protéines STOP chez les souris de type sauvage, montre une organisation parfaitement normale chez les souris KO -/-,
- l'examen du cortex somato-sensoriel, à partir de coupes tangentielles colorées pour mettre en évidence l'activité cytochrome oxydase, a montré une organisation normale des champs de tonneaux chez les souris KO -/-,
- le profil d'expression de la β-galactosidase dans le cerveau des souris STOP KO (-/-) est identique à celui observé chez les souris hétérozygotes ce qui démontre que les cellules qui, à l'état normal, expriment des quantités importantes de protéines STOP sont encore présentes chez les souris STOP -/-.

Les souris homozygotes STOP(-/-) qui ne présentent pas de lésions anatomiques du cerveau détectables en microscopie, présentent cependant des troubles du comportement.

### Exemple 2: Analyse électrophysiologique de la transmission synaptique chez les souris KO STOP -/-

### 1-Matériels et méthodes

Pour la préparation des coupes d'hippocampe, des souris âgées de 1 à 3 mois ont été profondément anesthésiées avec du nembutal. Des coupes de cerveau (300-400 µm) ont été réalisées dans un liquide céphalo-rachidien artificiel (124 mM NaCl, 26 mM NaHCO₃, 2,5 mM KCl, 1,25 mM NaH₂PO₄, 2,5 mM CaCl₂ et 1,3 mM MgCl₂), à une température comprise entre 4°C et 8°C. De manière plus précise : les coupes ont été maintenues à température ambiante pendant au moins 1 h puis elles ont été submergées dans une chambre contenant du liquide céphalo-rachidien artificiel équilibré avec 95 % d'O₂ et 5 % de CO₂ et transférées dans une chambre de superfusion.

Le potentiel d'excitation post-synaptique (EPSP) des champs extracellulaires a été enregistré à l'aide de microélectrodes (1 à 3 MΩ) remplies de liquide céphalo-rachidien artificiel. Les mesures ont été effectuées à une température d'environ 22 à 25°C. Des électrodes bipolaires en acier ont été utilisées pour stimuler la collatérale de Schaffer et la fibre moussue (stimulation de 10 à 100 mA, pendant 0,1 ms avec des intervalles de 10 à 30 s entre chaque stimulation).

Pour toutes les analyses effectuées dans la région CA1, les électrodes de stimulation et les électrodes de mesure extracellulaire ont été placées dans le *stratum radiatum* et de la picrotoxine (concentration finale de 100 µM, SIGMA) a été ajoutée au liquide céphalo-rachidien artificiel. Dans ces séries d'analyses, la région CA1 a été séparée de la région CA3 par section au couteau de la coupe de cerveau, avant la mesure.

Pour les courbes du type entrée-sortie, l'excitabilité des fibres a été analysée après blocage de l'activation du récepteur au glutamate par l'antagoniste du récepteur de l'AMPA (alpha-amino-3-hydroxy-5-méthyl-4-isoxazole acide propionique), NBQX (5 à 10 µM, TOCRIS). Les réponses du récepteur de l'AMPA ont été mesurées puis les réponses du récepteur du NMDA (N-méthyl-D-asparte) ont été révélées après suppression du magnésium extra-cellulaire et isolées par l'addition de NBQX (10 µM), afin d'effectuer une analyse quantitative.

Pour les analyses de type potentialisation post-tétanique (PTP) une dose élevée de D-APV (50 à 100 µM ; fourni par TOCRIS) a été ajoutée dans la solution du bain, au moins 10 minutes avant le choc tétanique.

Pour les expériences de stimulations couplées, les collatérales de Schaffer ont été stimulées de façon répétée par deux stimuli de même intensité séparés par de courts intervalles de durée variable. Le résultat est exprimé par le rapport entre l'amplitude de la réponse au second stimulus et au premier stimulus, déterminé à partir d'une moyenne de 15 à 20 réponses, pour chaque valeur de l'intervalle.

Les réponses des fibres moussues ont été analysées par l'application d'un bain de l'agoniste sélectif du récepteur au glutamate, DCG IV (groupe métabotropique de type 2). Les effets inhibiteurs du DCG IV (10 mM, fourni par TOCRIS) sur les entrées dans les fibres moussues sont similaires chez les souris de type sauvage et les souris STOP KO (-/-). NBQX (5 à 10 mM) a été appliqué à la fin de chaque analyse de la fibre moussue, afin de déterminer l'excitabilité de ces fibres.

L'acquisition des données et l'analyse des expériences de potentialisation à long terme (LTP) et de dépression à long terme (LTD) ont été effectuées en aveugle, relativement au génotype des souris. Tous les résultats sont exprimés sous forme de moyenne ± écart standard à la moyenne (s.e.m).

### 2- Résultats

Le fonctionnement des synapses des souris STOP KO (-/-) a été analysé dans l'hippocampe où l'expression des protéines STOP est importante.

Tout d'abord, afin d'analyser sélectivement la transmission glutamatergique, la transmission synaptique dans la région CA1 de l'hippocampe a été analysée en présence de picrotoxine, un antagoniste du récepteur du GABA de type A.

La transmission synaptique basale a été évaluée par l'analyse de la relation entre l'excitabilité des fibres des collatérales de Schaffer et l'amplitude des potentiels d'excitation post-synaptiques dans la région CA1 de l'hippocampe. L'analyse a été effectuée pour différentes intensités de stimulus. Les courbes du type entrée-sortie sont qualitativement similaires chez les souris STOP KO (-/-) et les souris de type sauvage (figures 5a et 5b). L'analyse quantitative effectuée sur les pentes des courbes entrée-sortie de six souris de type sauvage et de six souris KO STOP -/- ne montre aucune différence entre les 2 groupes de souris (figure 5c), indiquant une transmission synaptique basale normale chez les souris STOP -/-.

Pour l'analyse de la plasticité synaptique, la réponse synaptique à un stimulus standard est évaluée par la pente de la courbe d'EPSP. Les valeurs basales des pentes sont déterminées par des stimulations répétées à basse fréquence (0,03-0,1 Hz). Au temps zéro, un protocole de stimulation de conditionnement est appliqué. L'adaptation synaptique est mise en évidence par une déviation stable des valeurs des pentes d'EPSP, par rapport aux valeurs basales.

Un protocole de conditionnement à une fréquence élevée (100 Hz), appliqué à la synapse collatérale de Schaffer-cellule pyramidale de la région CA1, produit une augmentation stable des pentes des courbes, dans les coupes des souris de type sauvage (figure 6a), indiquant une potentialisation synaptique chez ces souris. Cette potentialisation a persisté plus de 30 minutes, et une telle persistance représente une potentialisation à long terme (LTP).

Chez les souris KO STOP (-/-) on observe une plus faible potentialisation de la transmission synaptique (figure 6b).

Cette différence est confirmée par l'analyse quantitative de l'ensemble des résultats obtenus dans les 2 groupes de souris (figure 6c).

La dépression à long terme (LTD) a été analysée au niveau des mêmes synapses des collatérales de Schaffer et des cellules pyramidales CA1. Le protocole classique de stimulation à basse fréquence a été utilisé (LFS, 900 stimulations de 1 Hz). Des coupes de souris STOP KO (-/-) ont montré une diminution significative de l'amplitude de LTD (figure 7b).

Ces résultats montrent que la LTP et la LTP sont altérées chez les souris STOP KO (-/-).

La LTP et la LTD dépendent de façon cruciale de l'activité du récepteur du NMDA. Toutefois, l'activité basale du récepteur du NMDA, mesurée par le rapport de la réponse NMDA/AMPA aux stimuli (figure 8A) et l'activation du récepteur NMDA pendant la stimulation tétanique (figure 8B) sont comparables chez les souris STOP KO (-/-) et les souris de type sauvage.

Ces résultats démontrent un déficit dans les 2 formes majeures de plasticité synaptique (LTP et LTD) chez les souris STOP KO (-/-) et indique que ce déficit n'est pas lié à un défaut d'expression du récepteur NMDA chez les souris STOP KO (-/-).

La figure 9B montre que chez les souris STOP KO (-/-), la potentialisation synaptique est altérée pendant les premières minutes après la stimulation tétanique ainsi qu'à des instants plus tardifs. En conséquence, l'existence d'un déficit éventuel de plasticité à court terme, au niveau des synapses des collatérales de Schaffer et des cellules pyramidales CA1, a été analysée par les mesures de la potentialisation post-synaptique (PTP) et de la facilitation par des stimulations couplées (PPF). Comme la LTP, la PTP est une forme de potentialisation suite à une stimulation tétanique (stimulus d' 1 Hz pendant 1 s) mais elle est induite en présence de l'antagoniste du récepteur NMDA (D-APV) pour bloquer des événements post-synaptiques impliqués dans la LTP, et persiste seulement pendant quelques minutes, suite à la stimulation tétanique. La PPF est une autre forme de plasticité synaptique observée lorsque les synapses sont stimulées par des stimulations couplées. La PPF est définie par une augmentation de la réponse synaptique en réponse au second stimulus. La PTP est réduite chez les souris STOP KO (-/-) (figure 9A). En revanche, la facilitation par stimulations couplées (PPF) est similaire chez les souris de type sauvage et les souris STOP KO (-/-) (figure 9B), et cela pour une gamme étendue de valeurs décroissantes de la concentration en calcium extracellulaire.

La plasticité synaptique au niveau des synapses des fibres moussues et des cellules pyramidales de la région CA3 a ensuite été analysée. Aucune différence dans la potentialisation à long terme (LTP) ainsi que dans la facilitation par stimulations couplées (PPF), n'a été observée entre les souris STOP KO (-/-) et les souris de type sauvage. Pour étudier la plasticité à court terme, les fibres moussues ont été stimulées par des fréquences croissantes allant de 0,033 à 1 Hz. Ce protocole induit normalement une augmentation importante et transitoire de l'amplitude de la réponse des fibres moussues, un phénomène dénommé facilitation de fréquence. L'amplitude de la facilitation de fréquence a été significativement diminuée chez les souris STOP KO (-/-), par comparaison avec les souris de type sauvage (figure 9C).

L'ensemble de ces résultats montre que plusieurs formes distinctes de la plasticité à long terme et à court terme sont altérées dans différentes régions de l'hippocampe, chez les souris STOP KO (-/-).

### Exemple 3 : Analyse des troubles du comportement des souris KO STOP -/-

### 1- Matériels et méthodes

Tous les tests de comportement ont été effectués sur des portées de souris STOP KO (-/-) et de souris témoins de type sauvage issues de la même colonie (fond génétique BALBc/129 Sv).

### 1-1 Tests du comportement maternel (maternage)

Le comportement maternel est apprécié par la réalisation des gestes suivants :
1. préparer un nid
2. réinstaller les nouveaux-nés dans le nid.

### Tests effectués chez les femelles nullipares et chez les mâles

De jeunes femelles nullipares âgées de 28 à 49 jours sont élevées individuellement pendant au moins un jour avant le début de l'expérience, puis du coton leur est fourni pour construire un nid.

A J1, chaque femelle est mise en présence de 3 nouveaux-nés âgés de 1 à 3 jours de la façon suivante : les nouveaux-nés sont placés chacun dans un des coins de la cage, à distance du nid et au bout de 30 minutes les nouveaux-nés sont replacés avec leur mère naturelle.

A J2, chaque femelle est à nouveau mise en présence des nouveaux-nés et on évalue, pour chaque femelle, le nombre de souriceaux réinstallés au nid pendant une période de 30 minutes.

Des jeunes mâles âgés de 30 à 45 jours ont été utilisés dans les mêmes conditions que les femelles nullipares à la seule différence qu'ils ont été mis en présence de souriceaux pendant 2 jours consécutifs avant d'être testés à J3, au lieu de J2 pour les femelles nullipares.

### Tests effectués chez les mères primipares ou multipares

Des femelles post-partum (seconde gestation) ont été élevées individuellement dès le début de leur gestation. Le jour de l'accouchement, les souriceaux ont été retirés et gardés au chaud pendant une heure. La mère a ensuite été retirée de sa cage habituelle et trois nouveaux-nés ont été placés chacun dans un coin de cette même cage, à distance du nid. Ensuite, la mère a été replacée dans son nid et le nombre de souriceaux réinstallés au nid pendant une période de 20 minutes, a été évalué.

### 1-2 "Test de lumière et d'obscurité"

Le test du choix entre la lumière et l'obscurité, dénommé "test de lumière et d'obscurité", est utilisé pour révéler un état d'anxiété provoqué par un stimulus générateur d'anxiété. Ce procédé, validé par Misslin et al. (1990, *Neuroreport,* 1, 267-270), repose sur la tendance naturelle des rongeurs à préférer un environnement sombre, et permet d'évaluer la réponse émotionnelle des animaux soumis à un stress constitué par la lumière.

Les animaux sont entretenus dans des cages individuelles placées dans un incubateur possédant une température comprise entre 21°C et 22°C et un cycle inversé lumière/obscurité de 12h/12h, avec de l'eau et de la nourriture, disponibles à volonté. Toutes les expériences sont réalisées conformément aux directives institutionnelles relatives à l'expérimentation animale.

Le dispositif consiste en deux boîtes de polyvinylcarbonate (20 cm x 20 cm x 14 cm) recouvertes de plexiglas. L'une des boîtes est obscurcie et l'autre boîte est éclairée à l'aide d'une lampe de bureau de 100 W placée à une distance de 15 cm (4400 lx). Un tunnel opaque en plastique (5 cm x 7 cm x 10 cm) sépare la boîte obscurcie de la boîte éclairée.

Les animaux sont placés individuellement dans la boîte éclairée avec la tête dirigée vers le tunnel. Le temps passé dans la boîte éclairée (TLB) et le nombre de passages entre les deux boîtes sont enregistrés sur une durée de 5 minutes, à partir de la première entrée des animaux dans la boîte obscurcie.

L'analyse globale des résultats est réalisée en utilisant le test U de Mann et Whitney. Le risque (p) est fixé à p <0,05. Les résultats sont exprimés par la valeur moyenne ± erreur standard à la moyenne (s.e.m).

### 1-3 Test de reconnaissance d'un objet

La mémoire à court terme est évaluée par le test de reconnaissance d'un objet précédemment décrit (Ennaceur et al., 1988, *Behav. Brain Res.,* 31, 47-59 ; Dodart et al., 1997, Neuroreport, 8, 1173-1178), qui repose sur la tendance naturelle des rongeurs à explorer un nouvel objet, de préférence à un objet familier.

Les animaux sont entretenus dans les conditions telles que décrites à l'exemple 3, section 1-2.

Le test de reconnaissance d'un objet est réalisé dans un espace ouvert en plexiglas (52 cm x 52 cm x 40 cm). Le sol est divisé en 9 carrés de taille égale. Les objets à distinguer sont une bille et un dé. Les animaux sont habitués au terrain ouvert pendant 30 min.

Le jour suivant, ils sont soumis à une épreuve d'apprentissage de 10 min (première épreuve) pendant laquelle ils sont placés individuellement dans l'espace ouvert, en présence d'un objet A (dé ou bille). Pendant cette période, sont enregistrés :
- l'activité locomotrice, évaluée par le nombre de carrés traversés, et
- le temps passé par l'animal à explorer l'objet A, c'est-à-dire le temps pendant lequel le nez de l'animal est dirigé à une distance de l'objet, inférieure à 1 cm.

Trois heures plus tard, ils sont soumis à une épreuve de reconnaissance de 10 min (deuxième épreuve). Pour cette épreuve, l'objet A et l'autre objet (B) sont placés dans l'espace ouvert et l'activité locomotrice, ainsi que la durée d'exploration de l'objet A (t_{A}) et de l'objet B (t_{B}) sont enregistrées. Ensuite; l'indice de reconnaissance (RI, en ordonnée à la figure 6) est calculé à partir de la formule suivante RI = t_{A}/(t_{A} + t_{B}) x 100. Un test de reconnaissance est considéré comme positif si la valeur de l'indice de reconnaissance est significativement supérieure à 50 %.

L'analyse globale des résultats est réalisée comme décrit à l'exemple 3, section 1-2.

### 1-4 Test de comportement social, test de l'intrus

### 1-4-1 Investigation sociale

Le comportement social est évalué sur de jeunes mâles âgés de 4 semaines, isolés pendant une semaine dans une cage (jeunes mâles résidents) ; un intrus mâle élevé en groupe est introduit dans la cage et le temps d'investigation sociale (approche, reniflement, attitude sexuelle) des jeunes mâles résidents est évalué pendant 6 minutes. L'analyse des résultats est réalisée en utilisant le test U Mann et Whitney.

Les résultats sont exprimés par la valeur moyenne ± erreur standard à la moyenne.

### 1-4-2 Agression inter-mâle

Des mâles résidents sont isolés pendant un mois et un intrus (mâle élevé en groupe) est placé dans la cage. On mesure le nombre d'attaques et le temps passé à se battre des résidents sur une période de 5 minutes. L'analyse des résultats est réalisée en utilisant le test U Mann et Whitney. Les résultats sont exprimés par la valeur moyenne ± une erreur standard à la moyenne.

### 2-Résultats

### 2-1 Comportement maternel des souris STOP KO (-/-)

Les souris STOP KO (-/-) présentent des défauts majeurs du comportement maternel se traduisant par une absence totale d'intérêt pour leur progéniture, comme le montrent les résultats présentés à la figure 10, obtenus à partir de 161 souriceaux issus de 20 souris femelles STOP KO (-/-) croisées avec des mâles hétérozygotes :
- tous les nouveaux-nés, issus de mères primipares STOP KO (-/-) meurent dans les 24 h suivant la naissance par défaut d'attention de la mère (figure 11a), et ce quel que soit leur génotype (fond génétique BALBc/129Sv ou 129Sv). Par comparaison, on observe un taux de survie de 93 % chez les nouveaux-nés issus de mère primipare portant l'allèle sauvage à l'état homozygote [souris sauvages (+/+)] qui présentent un comportement maternel normal comprenant en particulier la préparation d'un nid et la réinstallation des souriceaux dans le nid (figure 11a). Il a également été montré que le comportement maternel des souris STOP KO n'était pas amélioré par des gestations répétées (mères multipares).
- Les souriceaux nés de mère STOP KO (-/-) ne sont jamais cannibalisés et ils sont élevés jusqu'au sevrage lorsqu'ils sont adoptés par des mères de type sauvage, ce qui démontre que la mort des souriceaux est directement liée au génotype de la mère. Pour déterminer les causes de la mort, un défaut de tétée liée à une absence de signal olfactif au niveau des mamelles des femelles STOP KO (-/-) a été recherché. Lorsque les souriceaux issus de mère STOP KO (-/-) sont laissés en présence de leur mère mais replacés, de façon répétée, en position pour être maternés, par une intervention humaine, tous les souriceaux montrent un comportement de recherche et d'attachement à la mamelle. La présence de ce comportement guidé indique que les femelles STOP KO (-/-) possèdent les signaux olfactifs indispensables à l'allaitement. De plus, dans ces conditions, la présence de lait dans l'estomac des souriceaux a été observée. Ces résultats démontrent que la mort des souriceaux n'était pas liée à des défauts de lactation chez les souris STOP KO (-/-).
- Le défaut de réinstallation au nid n'est pas dû à un défaut de reconnaissance olfactive des souriceaux chez les souris STOP KO (-/-) étant donné que les femelles STOP KO (-/-) placées près de leur progéniture, reniflent les souriceaux et de plus montrent un comportement normal dans un test d'olfaction (test de la nourriture cachée).
- Afin de vérifier si le défaut de comportement maternel observé chez les souris STOP KO (-/-) était lié au statut hormonal, des tests complémentaires ont été effectués chez les femelles nullipares et chez les jeunes mâles. Les résultats des tests de réinstallation au nid, effectués chez ces deux groupes de souris montrent un défaut de comportement maternel des souris STOP KO (-/-) mâles ou femelles nullipares (figure 10b,et 10c).

L'ensemble de ces résultats indique que le défaut de comportement maternel observé chez les souris STOP KO (-/-) est indépendant d'un défaut organique manifeste et du statut hormonal de ces souris, ce qui indique qu'il n'est qu'une manifestation des défauts de comportement multiples observés chez ces souris STOP KO (-/-).

### 2-2 Autres troubles du comportement des souris STOP KO (-/-)

Alors que l'examen de l'état général des souris STOP KO (-/-) ne révèle aucun défaut apparent, elles présentent un comportement étrange avec des phases d'activité intense sans but apparent, accompagnées par de fréquents changements d'activité, au hasard. De manière occasionnelle, les souris présentent une période de crise, d'une durée d'environ 20 min, pendant laquelle les animaux effectuent des cercles ou creusent dans la cage, de manière compulsive. Ces souris traversent également des périodes de prostration apparente pendant lesquelles les souris restent immobiles, ne dorment pas et ne réagissent pas à l'environnement. De telles crises, n'ont jamais été observées chez les souris de type sauvage et ne ressemblent pas à des événements épileptiformes. Les crises aigües sont difficiles à étudier de façon systématique mais elles représentent des manifestations paroxystiques d'un bruit de fond continu d'anomalies du comportement. L'enregistrement vidéo a été utilisé pour évaluer le comportement des souris de façon quantitative. Le temps passé par les souris à manger, dormir, faire leur toilette, marcher et rester immobiles en étant toujours éveillées a été mesuré pendant une période de 3 heures, et les résultats sont présentés à la figure 11a. Par rapport aux souris de type sauvage, les souris STOP KO (-/-) passent plus de temps à circuler dans la cage ou à rester immobiles, alors qu'elles sont éveillées, au détriment du temps passer à se nourrir et à dormir. Les souris mutantes présentent des changements d'activités plus importants, en grande partie dus à un nombre significativement plus grand de phases de déplacement sans but et de phases d'immobilité (figure 11a). Les changements d'activité chez les souris STOP KO (-/-) rompent souvent une période d'activité caractéristique. Par exemple, chez les souris de type sauvage, 71% des phases de sommeil sont précédées d'une phase de toilette avec ou sans intercalage d'une phase de tranquillité. La fréquence correspondante chez les souris STOP KO (-/) est de 47 %, une valeur juste au dessus du bruit de fond attendu dans le cas de séquences d'activité au hasard (35 %, figure 12a). Ces résultats quantitatifs confirment l'impression d'activité non-organisée et non-orientée vers un but, donnée par l'observation des souris STOP KO (-/-).

Des analyses complémentaires du comportement des souris STOP KO (-/-) ont été effectuées à l'aide de tests classiques.

### Etat d'anxiété des souris STOP KO (-/-)

Les souris STOP KO (-/-) sont effrayées par un stimulus générateur d'anxiété et les souris sauvages (+/+) ont un comportement normal, comme le montrent les résultats du test de stimulation par la lumière (test lumière-obscurité) présentés aux figures 12a et 12b. En outre, il a été montré par des tests préalables, que l'activité locomotrice spontanée des souris mutantes (-/-) n'était pas modifiée comparativement aux souris sauvages (+/+).
- la figure 12a montre que les souris sauvages (+/+) passent beaucoup plus de temps dans la boîte éclairée que les souris STOP KO (-/-) qui restent dans la boîte obscurcie pendant presque toute la durée du test. Les différences observées entre les souris KO (-/-) et les souris sauvages (+/+) sont statistiquement significatives : p < 0,01, test U Mann et Whitney.
- la figure 12b montre que les souris sauvages entrent plus fréquemment dans la boîte éclairée que les souris STOP KO (-/-). Les différences observées entre les souris STOP KO (-/-) et les souris sauvages (+/+) sont statistiquement significatives : p < 0,01, test U Mann et Whitney.

### Mémoire à court terme des souris STOP KO (-/-)

Les souris STOP KO (-/-) présentent des troubles de la mémoire à court terme comme le montrent les résultats du test de reconnaissance, présentés à la figure 13 :
- l'indice de reconnaissance, mesuré chez les souris sauvages (65 %) est significativement plus élevé que l'indice de 50% observé chez les souris STOP KO (-/-) (p = 0,004 pour les souris (+/+), test de Student)..
- le temps d'exploration des objets A et B, enregistré lors de la deuxième épreuve (épreuve de reconnaissance) montre que les souris sauvages explorent le nouvel objet de préférence à l'objet familier.
- les souris STOP KO (-/-) n'explorent aucun des objets pendant les deux épreuves (apprentissage et reconnaissance). Elles se déplacent dans l'espace ouvert mais ne sont pas intéressées par les objets. Ce comportement pourrait s'expliquer par leur état d'anxiété comme le montrent les résultats du test de la lumière et de l'obscurité. Cet état d'anxiété est révélé notamment par le fait que les souris se déplacent le long des murs et qu'elles ne traversent pas l'espace ouvert, car elles semblent effrayées par l'environnement et par les objets qui représentent un stimulus générateur d'anxiété.

### Comportement social

Les souris STOP KO (-/-) présentent des troubles d'investigation sociale (figure 14a, 14b et 14c).
- La figure 14a montre que le temps passé par les résidents à explorer l'intrus est réduit de manière significative lorsque le résident est un mâle STOP KO (-/-), (p < 0,05).
- La figure 14b montre que dans le test d'agression inter-mâle, le nombre d'attaques effectuées par les résidents STOP KO (-/-) est inférieur au nombre d'attaques effectuées par les mâles sauvages (p < 0,01).
- La figure 14c montre que le temps passé à se battre par les résidents mâles STOP KO (-/-) est réduit en comparaison des mâles sauvages (p < 0,01).

### Exemple 4: Effet des anxiolytiques et des neuroleptiques sur le comportement maternel des souris STOP KO (-/-)

### 1-Matériels et méthodes

L'effet des anxiolytiques (diazépam) et des neuroleptiques (chlorpromazine, halopéridol ou clozapine), sur les troubles du comportement des souris STOP KO (-/-), a été évalué dans le test du comportement maternel tel que défini à l'exemple 3 (test de réinstallation au nid).

L'halopéridol (Haldol^{R}, JANSSEN-CILAG), la chlorpromazine (Largactil^{R}, RHONE-POULENC) et le diazépam (Valium^{R}, ROCHE) ont été administrés aux souris dans l'eau de boisson, à la dose de 0,5 mg/kg/jour.

### 2- Résultats

### a) traitement à court terme

L'effet de l'administration à court terme (pendant 6 à 8 jours à partir du 6^{ième} jour précédent l'accouchement) des anxiolytiques (diazépam) et des neuroleptiques (chlorpromazine, halopéridol ou clozapine) sur les troubles du comportement des souris STOP KO (-/-) a été évalué dans le test de réinstallation au nid.

La réinstallation des souriceaux au nid est dramatiquement altérée chez les mères STOP KO (-/-) non-traitées et est faiblement amélioré par l'administration de diazépam (figure 15A). En revanche, les mères STOP KO (-/-) traitées avec des neuroleptiques se comportent aussi bien que les souris de type sauvage (figure 15A). Cependant aucune survie des souriceaux n'a été observée aussi bien chez les souris STOP KO (-/-) traitées ou non-traitées. Ces résultats indiquent un effet bénéfique spécifique mais limité, de l'administration à court terme de neuroleptiques, sur le comportement des souris STOP KO (-/-).

### b) traitement à long terme

Sept souris STOP KO (-/-) et sept souris de type sauvage ont reçu une administration quotidienne d'un mélange de chlorpromazine et d'halopéridol, pendant 4 mois, à partir du sevrage et continuant pendant la croissance, l'accouplement avec les mâles, la gestation, l'accouchement et la période post-partum. Les sept souris de type sauvage ont présenté un comportement maternel normal et tous leurs souriceaux ont survécu (figure 15C). De façon remarquable, chez quatre des sept souris STOP KO (-/-), une amélioration du comportement maternelle a été suffisante pour permettre la survie des souriceaux, avec des rapports survivants/nouveaux-nés de respectivement 3/11, 4/8, 2/4 et 1/5, chez ces quatre souris.

La proportion de souris femelles STOP KO (-/-) avec des souriceaux survivants est significativement plus élevée chez les souris traitées à long terme avec des neuroleptiques (4/7), par rapport aux souris non-traitées (0/20) ou aux souris traitées à court terme avec des neuroleptiques (0/6, figure 15B).

Ces résultats indiquent que l'administration à long terme de neuroleptiques est capable de ré-établir un comportement normal chez les souris STOP KO (-/-), compatible avec la survie des souriceaux.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

## Revendications

1. Mammifère non-humain recombinant, porteur d'au moins un allèle modifié du gène codant pour une protéine STOP.

2. Mammifère non-humain recombinant selon la revendication 1, **caractérisé en ce que** ledit allèle modifié est obtenu à partir d'une construction comprenant une séquence d'acide nucléique issue du même mammifère ou d'un mammifère différent de celui dans lequel ladite construction est insérée, laquelle construction est sélectionnée dans le groupe constitué par des constructions contenant une séquence codant pour une protéine STOP anti-sens, des constructions comprenant la région du promoteur STOP en combinaison avec un gène rapporteur ou avec la région codante STOP, des constructions comprenant au moins une portion du gène STOP incluant au moins une modification, des constructions comprenant au moins une portion du gène STOP liée de manière opérationnelle à un promoteur et éventuellement à d'autres séquences régulatrices nécessaires pour l'expression dans l'animal hôte.

3. Mammifère non-humain recombinant selon la revendication 1 ou la revendication 2, **caractérisé en ce que** lesdites constructions sont sélectionnées parmi les constructions suivantes : des constructions qui incluent un fragment de la séquence génomique codant pour une protéine STOP comprise entre le codon d'initiation et le codon STOP, des constructions ne comprenant pas la région comprise entre les positions 4118 et 5131 de la séquence génomique codant pour une protéine STOP, des constructions comprenant 4,1 kb du gène STOP, le gène codant pour la β-galactosidase, placé sous le contrôle du promoteur STOP endogène, un gène de résistance à la néomycine sous le contrôle du promoteur PGK, 1,57 kb de séquence du gène STOP et enfin le gène de la thymidine kinase, sous le contrôle du promoteur PGK.

4. Utilisation d'un mammifère non-humain recombinant selon l'une quelconque des revendications 1 à 3, pour la sélection ou le criblage de produits psychoactifs.

5. Amorces ou sondes de criblage de clones ES recombinants ou d'animaux recombinants, **caractérisées en ce qu'**elles sont sélectionnées dans le groupe constitué par les séquences SEQ ID NO:3 à 6.

6. Procédé de criblage et de sélection de molécules utiles dans le traitement de la schizophrénie et des désordres schizoaffectifs à composante anxieuse, paranoïaque ou dépressive, lequel procédé est **caractérisé en ce qu'**il comprend au moins étapes suivantes :
- la mise en contact *in vitro* d'au moins une substance à cribler avec un échantillon biologique constitué par un extrait de cellules ou des tranches d'organes, de préférence neuronales ou cérébrales, obtenu à partir d'au moins un mammifère non-humain recombinant porteur d'au moins un allèle modifié du gène codant pour une protéine STOP selon l'une quelconque des revendications 1 à 3,
- la mesure de l'action de ladite substance à cribler sur lesdites cellules ou tranches d'organes et
- la comparaison des valeurs obtenues avec celles des cellules ou des tranches d'organes d'un échantillon biologique obtenu à partir d'un mammifère non-humain du même type, porteur de deux allèles sauvages du gène codant pour une protéine STOP.

7. Procédé selon la revendication 6, **caractérisé en ce que** ladite mesure est effectuée à l'aide d'un test de liaison protéine-protéine.

8. Procédé selon la revendication 6, **caractérisé en ce que** ladite mesure est effectuée par détection de la variation d'intensité d'un signal électrique.

9. Vecteur de recombinaison homologue d'un gène codant pour une protéine STOP, **caractérisé en ce qu'**il comprend une séquence nucléotidique d'un gène STOP modifié codant pour une protéine STOP inactivée, dont un fragment de 1012 pb contenant les séquences répétées de la région codante de l'exon 1 a été délétée et remplacée par une cassette d'expression contenant le gène de résistance à la néomycine (*néo*) sous le contrôle du promoteur PGK et le gène de la β-galactosidase (*lacZ*) sous le contrôle transcriptionnel du promoteur STOP endogène.

10. Utilisation d'une amorce ou d'une sonde selon la revendication 5, pour le criblage de cellules ES recombinantes non-humaines ou d'animaux non-humains recombinants.

## Claims

1. A recombinant non-human mammal carrying at least one modified allele of the gene encoding a STOP protein.

2. The recombinant non-human mammal as claimed in claim 1, **characterized in that** said modified allele is obtained from a construct comprising a nucleic acid sequence derived from the same mammal or from a mammal which is different from that into which said construct is inserted, which construct is selected from the group consisting of constructs containing a sequence encoding a STOP protein which is antisense, constructs comprising the region of the STOP promoter in combination with a reporter gene or with the STOP coding region, constructs comprising at least one portion of the STOP gene including at least one modification, and constructs comprising at least one portion of the STOP gene functionally linked to a promoter and, optionally, to other regulatory sequences required for expression in the host animal.

3. The recombinant non-human mammal as claimed in claim 1 or claim 2, **characterized in that** said constructs are selected from the following constructs: constructs which include a fragment of the genomic sequence encoding a STOP protein included between the initiation codon and the STOP codon, constructs which do not comprise the region between positions 4118 and 5131 of the genomic sequence encoding a STOP protein, and constructs comprising 4.1 kb of the STOP gene, the gene encoding β-galactosidase, placed under the control of the endogenous STOP promoter, a neomycin resistance gene under the control of the PGK promoter, 1.57 kb of sequence of the STOP gene and, finally, the thymidine kinase gene under the control of PGK promoter.

4. The use of a recombinant non-human mammal as claimed in any one of claims 1 to 3, for selecting or screening psychoactive products.

5. A probe or a primer for screening recombinant ES clones or recombinant animals, **characterized in that** it is selected from the group consisting of the sequences SEQ ID Nos. 3 to 6.

6. A method for screening and selecting molecules of use in the treatment of schizophrenia and schizoaffective disorders with a component of anxiety, paranoia or depression, which method is **characterized in that** it comprises at least the following steps:
- bringing at least one substance to be screened into contact, *in vitro,* with a biological sample consisting of an extract of cells or organ slices, preferably of neurons or brains, obtained from at least one recombinant non-human mammal carrying at least one modified allele of the gene encoding a STOP protein as claimed in any one of claims 1 to 3,
- measuring the action of said substance to be screened on said cells or organ slices, and
- comparing the values obtained with those of the cells or of the organ slices of a biological sample obtained from a non-human mammal of the same type, carrying two wild-type alleles of the gene encoding a STOP protein.

7. The method as claimed in claim 6, **characterized in that** said measurement is carried out using a protein-protein binding assay.

8. The method as claimed in claim 6, **characterized in that** said measurement is carried out by detection of the variation in strength of an electrical signal.

9. A vector for homologous recombination of a gene encoding a STOP protein, **characterized in that** it comprises a nucleotide sequence of a modified STOP gene encoding an inactivated STOP protein, of which a 1012 pb fragment, containing the repeat sequences of the coding region of exon 1 was deleted and replaced with an expression cassette containing the neomycin (*neo*) resistance gene under the control of the PGK promoter and the β-galactosidase (*lacZ*) gene under the transcriptional control of the endogenous STOP promoter.

10. The use of a primer or of a probe as claimed in claim 5, for screening recombinant non-human ES cells or recombinant non-human animals.

## Patentansprüche

1. Nicht-menschliches rekombinantes Säugetier, Träger wenigstens eines modifizierten Allels des Gens, das für ein STOP-Protein codiert.

2. Nicht-menschliches rekombinantes Säugetier nach Anspruch 1, **dadurch gekennzeichnet, dass** das genannte modifizierte Allel aus einer Konstruktion erhalten wird, die eine Nucleinsäuresequenz umfasst, die aus demselben Säugetier wie oder einem anderen Säugetier als das, in das die Konstruktion insertiert wird, stammt, wobei die Konstruktion ausgewählt ist aus der Gruppe, bestehend aus Konstruktionen, die eine Sequenz enthalten, welche für ein Antisense-STOP-Protein codiert; Konstruktionen, die die Region des STOP-Promotors in Kombination mit einem Reportergen oder mit der STOPcodierenden Region umfassen; Konstruktionen, die wenigstens einen Teil des STOP-Gens, der wenigstens eine Modifikation einschließt, umfassen; Konstruktionen, die wenigstens einen Teil des STOP-Gens funktionell an einen Promotor und gegebenenfalls an andere regulatorische Sequenzen, die für die Expression im Wirtstier erforderlich sind, gebunden umfassen.

3. Nicht-menschliches rekombinantes Säugetier nach Anspruch 1 oder Anspruch 2, **dadurch gekennzeichnet, dass** die genannten Konstruktionen unter den folgenden Konstruktionen ausgewählt sind: Konstruktionen, die ein Fragment der genomischen Sequenz, die für ein STOP-Protein codiert, zwischen dem Initiationscodon und dem STOP-Codon enthalten; Konstruktionen, die nicht die Region zwischen den Positionen 4118 und 5131 der genomischen Sequenz, die für ein STOP-Protein codiert, umfassen; Konstruktionen, die 4,1 kb des STOP-Gens, das Gen, das für die β-Galactosidase codiert, das unter die Kontrolle des endogenen STOP-Promotors gestellt ist, ein Gen für Resistenz gegen Neomycin unter der Kontrolle des PGK-Promotors, 1,57 kb der Sequenz des STOP-Gens und schließlich das Gen der Thymidinkinase unter der Kontrolle des PGK-Promotors umfassen.

4. Verwendung eines nicht-menschlichen rekombinanten Säugetiers nach einem der Ansprüche 1 bis 3 für die Auswahl oder die Durchmusterung von psychoaktiven Produkten.

5. Primer oder Sonden zur Durchmusterung von rekombinanten ES-Klonen oder rekombinanten Tieren, **dadurch gekennzeichnet, dass** sie aus der Gruppe, bestehend aus den Sequenzen SEQ ID NO: 3 bis 6, ausgewählt sind.

6. Verfahren zur Durchmusterung und Selektion von Molekülen, die in der Behandlung von Schizophrenie und schizoaffektiven Störungen mit Angst-, Paranoia-, oder Depressionskomponente verwendbar sind, wobei das Verfahren **dadurch gekennzeichnet ist, dass** es wenigstens die folgenden Stufen umfasst:
- in-vitro-Inkontaktbringen wenigstens einer durchzumusternden Substanz mit einer biologischen Probe, die aus einem Extrakt von Zellen oder Organschnitten, vorzugsweise Neuronalen oder Zerebralen gebildet wird, der/die aus wenigstens einem nicht-menschlichen rekombinanten Säugetier stammen, das Träger wenigstens eines modifizierten Allels des Gens, das für ein STOP-Protein nach einem der Ansprüche 1 bis 3 codiert, ist;
- Messen der Wirkung der genannten Substanz zur Durchmusterung der genannten Zellen oder Organschnitte und
- Vergleich der erhaltenen Werte mit denen der Zellen oder Organschnitte einer biologischen Probe, die von einem nicht-menschlichen Säugetier desselben Typs, das Träger von zwei Wildallelen des Gens, das für ein STOP-Protein codiert, ist, stammt.

7. Verfahren nach Anspruch 6, **dadurch gekennzeichnet, dass** die Messung mit Hilfe eines Protein-Protein-Bindungstests durchgeführt wird.

8. Verfahren nach Anspruch 6, **dadurch gekennzeichnet, dass** die genannte Messung durch Detektion der Intensitätsänderung eines elektrischen Signals durchgeführt wird.

9. Vektor zur homologen Rekombination eines Gens, das für ein STOP-Protein codiert, **dadurch gekennzeichnet, dass** er eine Nucleotidsequenz eines modifizierten STOP-Gens, das für ein inaktiviertes STOP-Protein codiert, umfasst, von dem ein Fragment mit 1012 bp, das die sich wiederholenden Sequenzen der Region, die für das Exon 1 codiert, enthält, deletiert und durch eine Expressionskassette ersetzt wurde, die das Gen für Neomycinresistenz (neo) unter der Kontrolle des PGK-Promotors und das Gen der β-Galactosidase (*lacZ*) unter der Transkriptionskontrolle des endogenen STOP-Promotors enthält.

10. Verwendung eines Primers oder einer Sonde nach Anspruch 5 zur Durchmusterung von nicht-menschlichen rekombinanten ES-Zellen oder von nicht-menschlichen rekombinanten Tieren.
